# EUROPÄISCHE PATENTSCHRIFT

(11) **EP 3 662 747 B1**
(45) Veröffentlichungstag und Bekanntmachung des Hinweises auf die Patenterteilung: **03.05.2023**
(21) Anmeldenummer: 19214080.4
(22) Anmeldetag: 06.12.2019
(51) Int. Cl.: A01K 11/00, A01K 29/00, A61B 5/00, A61B 5/08

(54) **VORRICHTUNG ZUR ERFASSUNG VON TIERPARAMETERN, SYSTEM ZUR ERFASSUNG UND AUSWERTUNG VON TIERPARAMETERN UND EIN COM-PUTERPROGRAMMPRODUKT ZUM MANAGEMENT EINER TIERHERDE**
DEVICE FOR DETECTING ANIMAL PARAMETERS, SYSTEM FOR DETECTING AND EVALUATING ANIMAL PARAMETERS, AND A COMPUTER PROGRAM PRODUCT FOR MANAGING AN ANIMAL HERD
DISPOSITIF DE DÉTECTION DE PARAMÈTRES D'ANIMAUX, SYSTÈME DE DÉTECTION ET D'ÉVALUATION DE PARAMÈTRES D'ANIMAUX ET PRODUIT PROGRAMME INFORMATIQUE PERMETTANT DE GÉRER UN TROUPEAU D'ANIMAUX

(30) Priorität: 07.12.2018 DE 202018107000 U
(43) Veröffentlichungstag der Anmeldung: 10.06.2020
(73) Patentinhaber: Leibniz-Institut für Agrartechnik und Bioökonomie e.V. (ATB), 14469 Potsdam (DE); Fiske, Daniel, 14476 Potsdam (DE)
(72) Erfinder: Fiske, Daniel, 14476 Potsdam (DE); Strutzke, Saskia, 14476 Potsdam (DE)
(74) Vertreter: Hertin und Partner Rechts- und Patentanwälte PartG mbB

(56) Entgegenhaltungen:
- EP-A1- 3 335 549
- WO-A1-2017/096256
- US-A1- 2007 107 668
- US-A1- 2018 325 382
- STRUTZKE S ET AL: "Technical note:Development of a noninvasive respiration rate sensor for cattle", JOURNAL OF DAIRY SCIENCE, vol. 102, no. 1, 8 November 2018 (2018-11-08), pages 690-695, XP085563752, ISSN: 0022-0302, DOI: 10.3168/JDS.2018-14999

## Beschreibung

Die Erfindung betrifft eine Vorrichtung zur Erfassung von Tierparametern, wobei die Vorrichtung ein Sensorpaket mit einem oder mehreren Sensoren zur Erfassung der Tierparameter umfasst. In weiteren Aspekten betrifft die Erfindung ein System zur Erfassung und Auswertung von Tierparametern, sowie ein Computerprogrammprodukt zum Management einer Tierherde.

### Stand der Technik:

Die Tiergesundheit und das Wohlbefinden von Tieren rückt in letzter Zeit vermehrt in den Fokus des Interesses der Öffentlichkeit. In Zeiten von aufgeklärten Verbrauchern wird der Ruf nach gesunden Nahrungsmitteln, insbesondere auch nachhaltig erzeugten tierischen Produkten, wie Fleisch, Milch und Eiern, immer lauter. Es besteht daher ein großes gesellschaftliches Interesse, das Wohlbefinden von Tieren in der Nutztierhaltung, insbesondere auch in der Massen-Nutztierhaltung, im Blick zu haben und den so gehaltenen Tieren ein - gemessen an den Umständen - möglichst erträgliches Leben zu ermöglichen.

Dazu wäre es wünschenswert, wenn Vorrichtungen und Verfahren bereitstehen würden, mit denen das Wohlbefinden beziehungsweise Parameter, die das Wohlbefinden eines Tieres beschreiben, erfasst werden könnte(n), wobei die Tiere durch die Erfassung dieser Daten möglichst wenig in ihrem normalen Verhalten und Alltagsleben gestört und beeinträchtigt werden sollen.

Es hat sich herausgestellt, dass insbesondere die Atemfrequenz ein wichtiger Parameter ist, um Rückschlüsse auf das Wohlbefinden eines Tieres zu ziehen. Bisher wird die Atemfrequenz bei Tieren, insbesondere bei landwirtschaftlich genutzten Großtieren, wie Kühen oder Rindern, oder aber auch bei Pferden, visuell bestimmt. Dabei wird das Tier beobachtet und die Anzahl der äußerlich wahrnehmbaren Atembewegungen des Tieres in Relation zu einer bestimmten Zeitspanne gesetzt. Unter dem Begriff der Atembewegung wird dabei beispielsweise das Heben und Senken des Brustkorbes oder des Bauchraumes eines Tieres verstanden. Dieses bisher praktizierte Verfahren ist sehr zeit- und personalaufwändig und darüber hinaus überaus anfällig für Fehler und Messungenauigkeiten. Die konventionelle Methode der visuellen Bestimmung der Atemfrequenz ist ferner nicht für eine Daueranwendung geeignet, um die Atemfrequenz, beispielsweise als Indikator für das Wohlbefinden oder ein Stresslevel des Tieres, über einen längeren Zeitraum zu beobachten. Es sind im Stand der Technik auch Verfahren bekannt, bei denen die Tiere narkotisiert werden müssen oder bei denen aufwändige experimentelle Apparaturen verwendet werden müssen, um die Atemfrequenz des Tieres zu bestimmen.

Strutzke al., J. Dairy Sci. 102 (2018), S. 690-695, offenbaren eine Vorrichtung zur Erfassung von Tierparametern mittels eines Druckdifferenzsensors.

Es ist daher Aufgabe der vorliegenden Erfindung, Vorrichtungen und Systeme zur Erfassung von Tierparametern, beispielsweise der Atemfrequenz, bereitzustellen, die nicht die Mängel und Nachteile des Standes der Technik aufweisen. Dabei sollen das System und die Vorrichtung bei der Erfassung der Parameter die Tiere möglichst wenig in ihrer Bewegungsfreiheit und ihrem natürlichen Verhalten einschränken, ihnen keine Schmerzen verursachen oder anderweitige Schäden zuführen, leicht anzubringen und zu entfernen und tierindividuell anpassbar sein.

### Beschreibung der Erfindung:

Gelöst wird die Aufgabe durch die Merkmale der unabhängigen Ansprüche. Vorteilhafte Ausgestaltungen der Erfindung sind in den abhängigen Ansprüchen beschrieben. Erfindungsgemäß ist eine Vorrichtung zur Erfassung von Tierparametern vorgesehen, wobei die Vorrichtung ein Sensorpaket mit einem oder mehreren Sensoren zur Erfassung der Tierparameter umfasst, wobei das Sensorpaket mindestens einen Druckdifferenzsensor sowie einen Feuchtesensor umfasst, wobei die Vorrichtung am Kopf und/oder am Hals eines Tieres anbringbar ist, wobei aus den erfassten Tierparametern Rückschlüsse auf einen Zustand der Tieres ableitbar sind, wobei unterschiedliche Zustände des Tieres mit einer zustandsspezifischen Kennzeichnung an der Vorrichtung anzeigbar sind.

Es ist im Sinne der Erfindung bevorzugt, dass die Vorrichtung als Erfassungsvorrichtung bezeichnet wird. Bei den Tieren, bei denen die Erfindung angewendet werden kann, handelt es sich beispielsweise um Kühe, insbesondere Rinder, Pferde oder Hunde. Es ist allerdings auch vorstellbar, dass die Erfindung in Verbindung mit Schweinen oder in Gehegen gehaltenen Paarhufern, wie Rehen, angewendet wird. Bei den mit der vorgeschlagenen Vorrichtung zu erfassenden Tierparametern kann es sich beispielsweise um die Atemfrequenz des Tieres, sein Atemverhalten, sein Wiederkäuverhalten, Lautäußerungen, Ausatmungsfeuchte, Körpertemperatur, Puls, Herzfrequenz und/oder Stoffwechselgase handeln, ohne darauf beschränkt zu sein. Die Stoffwechselgase können zum Beispiel für die vorteilhafterweise für die Ketose-Erkennung verwendet werden. Es ist im Sinne der Erfindung bevorzugt, dass die Vorrichtung diese Tierparameter mit den Sensoren des Sensorpakets detektiert und/oder erfasst. Erfindungsgemäß kann die Vorrichtung am Kopf und/oder am Hals eines Tieres angebracht werden, wobei die Vorrichtung dazu eingerichtet ist, aus den erfassten Tierparametern Rückschlüsse auf einen Zustand des Tieres abzuleiten, wobei eine zustandsspezifische Kennzeichnung an der Vorrichtung dazu eingerichtet ist, unterschiedliche Zustände des Tieres anzuzeigen. Dazu kann vorzugsweise eine optische Anzeige verwendet werden.

Vorteilhafterweise können die Tierparameter mit der Erfassungsvorrichtung tierindividuell ermittelt werden, wobei die Tiere vorzugsweise einzeln mit dem System beziehungsweise der Vorrichtung identifiziert werden können. Die Erfindung betrifft daher eine Vorrichtung zur individuellen Erfassung von Tierparametern. Vorzugsweise können mehrere der Erfassungsvorrichtungen zusammen verwendet werden, beispielsweise wenn jedes Tier einer Herde oder eine Teilmenge von Tieren einer Herde mit den Erfassungsvorrichtungen ausgestattet ist. Die Vorrichtungen sind vorzugsweise aus der Ferne programmierbar und die Tiere können einzeln durch eine optische Anzeige gekennzeichnet werden. Der Begriff "kennzeichnen" bedeutet im Sinne der Erfindung bevorzugt, dass die Vorrichtung eines Tieres in einer bestimmten Farbe aufleuchtet oder blinkt, wenn das Tier ein bestimmtes Verhalten zeigt oder wenn die von den Sensoren erfassten Tierparameter eine Abweichung von einem Norm- und/oder Vergleichswert anzeigen. Es ist vorteilhafterweise auch möglich, dass ein Nutzer, ein Herdenmanager und/oder ein Besitzer der Tiere die Tiere von seinem Arbeits-PC aus kennzeichnet, beispielsweise wenn eine neue Herde zusammengestellt werden soll oder einzelne Tiere zur Schlachtung oder zur Besamung separiert werden sollen.

Insofern betrifft die Erfindung vorzugsweise auch ein System zur Kennzeichnung einzelner Tiere aus einer Herde, wobei diese Kennzeichnung insbesondere aus der Ferne erfolgen können soll. Das System zur Kennzeichnung einzelner Tiere aus einer Herde umfasst eine oder mehrere Erfassungsvorrichtungen, sowie eine PC-Vorrichtung und/oder ein mobiles Endgerät, wobei je ein Tier einer Tierherde eine Erfassungsvorrichtung trägt. Das System zur Kennzeichnung einzelner Tiere aus einer Herde ist vorzugsweise dadurch gekennzeichnet, dass ein Nutzer ausgewählte Tiere einer Herde an der PC-Vorrichtung und/oder dem mobilen Endgerät auswählen kann, wobei durch die Auswahl des Nutzers ein Signal an die Erfassungsvorrichtung der ausgewählten Tiere gesendet wird, wobei das Signal dazu führt, dass eine optische Anzeige aktiviert wird. Es ist mit anderen Worten im Sinne der Erfindung bevorzugt, dass durch die Auswahl des Nutzers ein Signal an die Erfassungsvorrichtung der ausgewählten Tiere gesendet wird, wobei durch das Signal eine Aktivierung einer optischen Anzeige erfolgt, wodurch das Tier gekennzeichnet und damit von den anderen, nicht ausgewählten Tieren unterscheidbar gemacht wird. Dies wird beispielsweise dadurch erreicht, dass die optische Anzeige der Erfassungsvorrichtung des ausgewählten Tieres in einer bestimmten Farbe aufleuchtet oder blinkt, während die optische Anzeige der Erfassungsvorrichtung eines nicht ausgewählten Tieres nicht aufleuchtet oder blinkt.

Es stellt einen besonderen Vorteil der Erfindung dar, dass mit der vorgeschlagenen Vorrichtung und dem vorgeschlagenen System Gesundheits- und/oder Verhaltensparameter eines Tieres tierindividuell, automatisch, kontinuierlich und ortsunabhängig bestimmt und ausgewertet werden können und ferner Besonderheiten eines Tieres mit einer optischen Anzeige, die vorzugsweise in oder an der Erfassungsvorrichtung angeordnet ist, angezeigt werden können. Der Begriff "tierindividuell" bedeutet im Sinne der Erfindung bevorzugt, dass vorzugsweise jedes Tier einer Herde eine Erfassungsvorrichtung trägt, so dass die genannten Tierparameter für jedes der Tiere der Herde individuell ermittelt und ausgewertet werden können. Vorzugsweise werden die Daten von der Erfassungsvorrichtung an eine PC-Vorrichtung übertragen, wobei die ermittelten Tierparameter-Daten durch die PC-Vorrichtung ausgewertet werden können. Vorzugsweise erfolgt die Auswertung dieser Daten für jedes Tier einzeln, wobei es im Sinne der Erfindung auch bevorzugt sein kann, die Daten der einzelnen Tiere miteinander zu vergleichen beziehungsweise diese mit statistischen Methoden auszuwerten, beispielsweise um Vergleichsgrößen und/oder Normwerte zu bestimmen. Es ist im Sinne der Erfindung auch bevorzugt, dass die PC-Vorrichtung über Speichermittel verfügt, um die ermittelten Tierparameter-Daten vorzugsweise getrennt nach Tieren abzuspeichern. Es ist im Sinne der Erfindung auch bevorzugt, mehrere Tiere als Herden zusammen zu fassen und mit dem vorgeschlagenen System eine Herde oder mehrere Herden von Tieren zu managen und/oder zu verwalten. Dies kann beispielsweise vorteilhaft sein und die tägliche Arbeit wesentlich erleichtern, wenn ein größerer landwirtschaftlicher Betrieb über mehrere Ställe verfügt, in denen sich jeweils eine Tierherde aufhält.

Der Begriff "automatisch" bedeutet im Sinne der Erfindung bevorzugt, dass die Vorrichtung und das System vorzugsweise kontinuierlich arbeiten und nicht ein- oder ausgeschaltet werden müssen. Vielmehr stellt es einen wesentlichen Vorteil der Erfindung dar, dass mit der Erfindung eine Langzeit- oder Dauerüberwachung von Tieren ermöglicht wird, die für die Tiere besonders schonend und gut zu ertragen ist. Tests haben gezeigt, dass sich das Verhalten von Tieren, die eine Erfassungsvorrichtung tragen, nicht wesentlich von solchen Tieren unterscheidet, die keine Erfassungsvorrichtung tragen. Insofern ermöglicht es die Erfindung insbesondere, besonders unverfälschte und realitätsgetreue Daten und Verhaltensmuster der Tiere aufzuzeichnen und dadurch Erkenntnisse und Zwischenergebnisse zur Optimierung eines Herdenmanagements zu erhalten.

Der Begriff "ortsunabhängig" bedeutet im Sinne der Erfindung bevorzugt, dass die Erfassungsvorrichtung mobil ausgebildet ist und insbesondere am Tier getragen werden kann. Dazu wird die Vorrichtung am Kopf und/oder am Hals eines Tieres angebracht. Dies kann beispielsweise in Form eines Nasenrings oder eines Halsrings erfolgen, wobei ein Halsring im Sinne der Erfindung bevorzugt auch als Halsband bezeichnet werden kann. Mit anderen Worten ist es im Sinne der Erfindung bevorzugt, dass ein Tier mit einer Erfassungsvorrichtung ausgestattet werden kann und mit dieser Erfassungsvorrichtung sein übliches Leben gestaltet. Dabei ist die vorgeschlagene Erfassungsvorrichtung insbesondere so ausgebildet, dass das Tier möglichst wenig in seinem Alltag gestört und/oder beeinträchtigt wird. Die Erfassungsvorrichtung ist insbesondere möglichst leicht und kompakt ausgebildet, so dass das Tier weiterhin gut fressen, trinken und atmen kann.

Ein besonderer Vorteil der Erfindung besteht darin, dass der vorgeschlagene Nasenring nicht-invasiv verwendet wird und insbesondere nicht die Nasenscheidewand des Tieres durchstößt. Es kann im Sinne der Erfindung auch bevorzugt sein, dass die Erfassungsvorrichtung als Vorrichtung ausgebildet ist, die an oder in der Nasengegend des Tieres angeordnet ist. Insbesondere kann die Erfassungsvorrichtung, vorzugsweise wenn die Verwendung an einem Pferd, an einer Kuh oder an einem Rind gewünscht wird, als Stent ausgebildet sein, der vorzugsweise ein biegsames und flexibles Material umfasst. Die Vorrichtung kann in dieser bevorzugten Ausführungsform der Erfindung in einem zusammengefalteten Zustand in eine oder beide Nasenöffnungen des Tieres eingeführt werden, wo es sich dann automatisch öffnet, d.h. entfaltet. Vorteilhafterweise kann sich die Vorrichtung selbständig der Form des Naseninnenraumes des Tieres anpassen. Diese speziell für Pferde entwickelte Ausführungsform für Pferde ist mit dem Vorteil verbunden, dass die Vorrichtung durch die Ausbildung als Stent innerhalb des Tieres getragen wird und dadurch nicht oder nur schwer von dem Tier abgestreift werden kann. Es ist im Sinne der Erfindung bevorzugt, dass die Erfassungsvorrichtung wie ein Stent in der Nase befestigt wird. Dadurch wird es vorteilhafterweise möglich, das Sensorpaket innerhalb der Nase des Tieres zu befestigen, so dass es praktisch für das Tier nicht möglich ist, die Erfassungsvorrichtung selbständig zu entfernen.

Dies kann auch dadurch erreicht werden, dass eine ringartig ausgebildete Vorrichtung mit einer Spange und/oder einem Clip an den Nüstern des Pferdes befestigt wird. Mit anderen Worten kann es im Sinne der Erfindung bevorzugt sein, dass die Vorrichtung ein Befestigungsmittel umfasst, das vorzugsweise als Spange und/oder einem Clip ausgebildet ist und dazu eingerichtet ist, die Vorrichtung an den Nüstern eines Tieres, vorzugsweise eines Pferdes, zu befestigen. Es ist im Sinne der Erfindung insbesondere bevorzugt, dass die Erfassungsvorrichtung ein mindestens ein Befestigungsmittel umfasst, wobei das Befestigungsmittel als Spange oder Clip ausgebildet ist. Vorzugsweise ist die Vorrichtung mit den Befestigungsmitteln an einem Nasenflügel und/oder Nüstern des Tieres anbringbar ist, wobei die Haltebacken in dieser bevorzugten Ausführungsform der Erfindung nicht an der Nasenscheidewand des Tieres anliegen, sondern an mindestens einem Nasenflügel oder den Nüstern des Tieres. Insbesondere wenn die Vorrichtung beziehungsweise das vorgeschlagene System an einem Pferd verwendet wird, kann es im Sinne der Erfindung auch bevorzugt sein, dass die Vorrichtung dazu eingerichtet ist, unterstützend beim Training eingesetzt zu werden. Beispielsweise kann die Vorrichtung zur Kontrolle und/oder Überwachung der Atmung und/oder des Pulses verwendet werden, um beispielsweise einen Trainings- oder Fitnesszustand des Tieres zu ermitteln.

Es ist ferner bevorzugt, die Erfassungsvorrichtung aus einem Kunststoffmaterial herzustellen, damit das Material der Vorrichtung vom Tier nicht als kalt empfunden wird. Die Ortsunabhängigkeit der Erfassungsvorrichtung wird vorzugsweise dadurch unterstützt, dass die durch das Sensorpaket der Erfassungsvorrichtung ermittelten Daten, vorzugsweise die erfassten Tierparameter, drahtlos an eine Auswertungseinheit übermittelt werden können. Dazu umfasst die Erfassungsvorrichtung vorzugsweise geeignete Kommunikationsmittel, die vorzugsweise eine drahtlose Kommunikation zwischen Erfassungsvorrichtung und Auswertungseinheit ermöglichen. Es ist im Sinne der Erfindung ganz besonders bevorzugt, dass eine Auswertung der erfassten Tierparameter in einer Auswerteeinheit der PC-Vorrichtung erfolgt. Es kann im Sinne der Erfindung bevorzugt sein, dass die PC-Vorrichtung ein programmierbarer Computer, ein Computer, ein Gerät zur Datenverarbeitung, ein universeller Computer, beispielsweise ein Laptop, eine Smartwatch, ein Handy, ein PC, ein Mikrokontroller oder ein Großrechner ist, ohne darauf beschränkt zu sein. Es kann im Sinne der Erfindung auch bevorzugt sein, dass Auswertung der erfassten Tierparameter in der Erfassungsvorrichtung selbst erfolgt. Dies kann beispielsweise durch einen Microcontroller geschehen, der vorzugsweise Bestandteil der Erfassungsvorrichtung sein kann.

Es war vollkommen überraschend, dass mit der vorgeschlagenen Vorrichtung und dem vorgeschlagenen System aus den erfassten Tierparametern Rückschlüsse auf einen Zustand eines Tieres abgeleitet werden können. Beispielsweise kann die Atemfrequenz des Tieres dazu verwendet werden, Rückschlüsse auf das gesamte Atemverhalten des Tieres zu ziehen, wobei das Ziehen von Rückschlüssen im Sinne der Erfindung bevorzugt als "Auswertung der ermittelten Tierparameter" bezeichnet wird. Das Atemverhalten des Tieres kann beispielsweise durch Atemaussetzer, Husten, die Einatmungstiefe, die vorzugsweise durch eine Amplitude beschrieben werden kann, oder eine Veränderung der Lungenkapazität des Tieres charakterisiert werden.

Insbesondere ermöglicht das vorgeschlagene System und die vorgeschlagene Erfassungsvorrichtung eine Verhaltensaufzeichnung von Tieren für Langzeitstudien.

Diese können insbesondere zur Identifizierung von stressverursachenden Faktoren im Bereich der Tierhaltung verwendet werden, denn die Erfinder haben erkannt, dass ein Zusammenhang besteht zwischen Entspannungs- beziehungsweise Stresszustand eines Tieres und der Ausprägung seiner Atemfrequenz, die aus den erhobenen Tierparametern ermittelbar ist. Überraschenderweise kann mit dem vorgeschlagenen System nicht nur die Atmung, sondern es können auch Lautäußerungen, wie zum Beispiel Muhen, das Gemolkenwerden, Stressphasen oder auch die Futtersuche klar von anderen Verhaltensmerkmalen unterschieden werden. Beispielsweise können bei Einsatz des vorgeschlagenen Systems und der Vorrichtung auch verschiedene Tätigkeiten oder Verhaltensweisen, wie Liegen, Stehen oder Laufen, voneinander unterschieden werden. Dadurch können Vorgänge im Stall, beim Melken oder allgemein im Umgang mit dem Tier vorteilhafterweise optimiert werden.

Es ist im Sinne der Erfindung vorgesehen, dass die vorgeschlagene Erfassungsvorrichtung einen Druckdifferenzsensor umfasst. Dieser kann vorzugsweise in einer oder beiden Nasenöffnungen des Tieres angeordnet vorliegen und er ist bevorzugt dazu eingerichtet, eine Druckdifferenz zwischen dem Druck innerhalb der Nasenöffnung und einem Druck außerhalb der Nasenöffnung, der vorzugsweise als Atmosphärendruck oder Umgebungsdruck bezeichnet wird, zu messen. Es ist im Sinne der Erfindung bevorzugt, aus den ermittelten Druckwerten und/oder den Druckdifferenzen eine Atemfrequenz eines Tieres zu bestimmen. Insbesondere wird es mit dem System und der Erfassungsvorrichtung ermöglicht, das Atemverhalten eines Tieres zu untersuchen und/oder zu analysieren. Der Begriff der Atmung umfasst im Sinne der Erfindung bevorzugt die Atemfrequenz, das Atemmuster, Abweichungen von festgestellten Atemmustern oder weitere Parameter, die mit der Atmung in Verbindung stehen. Tests haben gezeigt, dass das System und das Verfahren vorteilhafterweise dazu verwendet werden können, ein Ruheverhalten und/oder einen Entspannungszustand eines Tieres deutlich von einem Aktivitäts- und/oder Stressverhalten zu unterscheiden. Neben der Atmung kann es im Sinne der Erfindung auch bevorzugt sein, das Wiederkauverhalten eines Tieres mit dem vorgeschlagenen System oder dem vorgeschlagenen Verfahren zu analysieren.

Es stellt einen weiteren besonderen Vorteil der Erfindung dar, dass unterschiedliche Zustände des Tieres mit einer zustandsspezifischen Kennzeichnung an der Vorrichtung anzeigbar sind. Bei den Zuständen des Tieres kann es sich um unterschiedliche Aspekte des Gesundheits- und/oder Verhaltensparameter eines Tieres handeln, beispielsweise seine Brunft, sein Atemverhalten, seine Körpertemperatur oder beispielsweise seine Vorsehung zur Schlachtung. Es ist im Sinne der Erfindung bevorzugt, dass die einzelnen Zustände binäre Ausprägungen aufweisen können. Dies ist beispielsweise bei der Vorsehung des Tieres zur Schlachtung der Fall, denn entweder ist das Tier zur Schlachtung vorgesehen oder es ist nicht zur Schlachtung vorgesehen. Eine solche binäre Ausprägung kann beispielsweise auch der Körpertemperatur oder dem Puls des Tieres zugeordnet werden, indem beispielsweise die ermittelten Daten einem Normbereich zugeordnet werden, der vorzugsweise als "nicht auffällig" bezeichnet werden kann, und indem andere ermittelte Daten als Abweichung von einer Norm- und/oder Vergleichswert eingeordnet werden können. Es kann im Sinne der Erfindung auch bevorzugt sein, dass einzelne Zustände graduelle Ausprägungen aufweisen, wie beispielsweise die Brünstigkeit des Tieres, dem beispielsweise Stufen "nicht brünstig", "schwach brünstig", "stark brünstig" zugeordnet werden können. Der Begriff "zustandsspezifisch" bedeutet im Sinne der Erfindung bevorzugt, dass mit der vorgeschlagenen Erfassungsvorrichtung beziehungsweise dem vorgeschlagenen System eine binäre oder graduelle Ausprägung eines Zustands eines Tieres angezeigt werden kann.

Die zustandsspezifische Kennzeichnung wird im Sinne der Erfindung bevorzugt durch eine optische Anzeige, die vorzugsweise Bestandteil der Erfassungsvorrichtung ist, angezeigt. Es ist im Sinne der Erfindung insbesondere bevorzugt, dass die Erfassungsvorrichtung ein Leuchtmittel zur Kennzeichnung der unterschiedlichen Zustände des Tiers umfasst, wobei das Leuchtmittel vorzugsweise zur optischen Anzeige der unterschiedlichen Ausprägungen der Zustände des Tieres verwendet wird. Es ist im Sinne der Erfindung bevorzugt, dass die Anzeige eines Zustands des Tieres durch die optische Anzeige erfolgt, wobei es sich bei der optischen Anzeige zum Beispiel um ein Leuchtmittel aufweisend eine oder mehrere *light emitting dio*des (LEDs) handeln kann. Es ist im Sinne der Erfindung insbesondere bevorzugt, dass die LEDs vorzugsweise in unterschiedlichen Farben leuchten können. Die Formulierung, dass "die LEDs in unterschiedlichen Farben leuchten" bedeutet im Sinne der Erfindung bevorzugt, dass die optische Anzeige Licht in unterschiedlichen Farben, d.h. Längenwellen- beziehungsweise Frequenzbereichen abgeben kann. Es ist im Sinne der Erfindung bevorzugt, dass die optische Anzeige im Wesentlichen im Dauerbetrieb arbeitet und dass sich beispielsweise die Farbe des abgegebenen Lichts ändert, wenn der Nasen- oder Halsring ein Signal von der Auswertungseinheit des Systems enthält. Es kann im Sinne der Erfindung ebenso bevorzugt sein, dass die optische Anzeige nicht im Dauerbetrieb arbeitet, sondern nur bei einer Aktivierung.

Vorzugsweise kann die Auswertung der ermittelten Tierparameter-Daten auf einer PC-Vorrichtung, der Erfassungsvorrichtung selbst oder auf einem mobilen Endgerät erfolgen, wobei die Einheit, auf der die Auswertung erfolgt, im Sinne der Erfindung vorzugsweise als Auswertungseinheit bezeichnet wird. Es ist im Sinne der Erfindung ganz besonders bevorzugt, dass unterschiedliche Zustände des Tieres mit unterschiedlichen Farben gekennzeichnet werden. Es ist im Sinne der Erfindung darüber hinaus bevorzugt, dass die unterschiedlichen Ausprägungen der Zustände eines Tieres mit unterschiedlichen Farbausprägungen, wie Intensität des abgegebenen Lichts oder einem variierenden Farbton, markiert werden. Diese Markierung von unterschiedlichen Zuständen des Tieres beziehungsweise der unterschiedlichen Ausprägungen der Zustände eines Tieres durch unterschiedliche Farben oder Farbausprägungen wird im Sinne der Erfindung bevorzugt als "zustandsspezifische Kennzeichnung" bezeichnet.

Die zustandsspezifische Kennzeichnung stellt einen besonderen Vorteil der Erfindung dar, wie in den folgenden Anwendungsbeispielen dargestellt wird. Dabei werden sowohl Vorteile der vorgeschlagenen Erfassungsvorrichtung genannt, als auch die Vorteile eines Systems zur Erfassung und Auswertung von Tierparametern und eines Computerprogrammprodukts zum Management einer Tierherde, wie sie weiter unten im Text vorgestellt werden.

Das vorgeschlagene System beziehungsweise die vorgeschlagene Erfassungsvorrichtung sind vorzugsweise in der Lage, die Tiere zu kennzeichnen. Es ist im Sinne der Erfindung bevorzugt, dass die Kennzeichnung ortsunabhängig von einem mobilen Endgerät und/oder von einer Feststation erfolgt. Bei der Feststation kann es sich beispielsweise um die vorzugsweise stationär, d.h. an einem bestimmten, festen Ort angeordnete PC-Vorrichtung handeln. Der Begriff "ortsunabhängig" bedeutet in diesem Zusammenhang bevorzugt, dass das mobile Endgerät eines Nutzers beziehungsweise der Nutzer selbst an einem anderen Ort sein kann als das Tier beziehungsweise die Tierherde. Dadurch kann ein Nutzer die Betreuung und/oder Versorgung eines Tieres oder einer Tierherde auch dann sicherstellen, wenn er physisch nicht anwesend ist. Insbesondere kann der Nutzer eine Tierherde von *remote* aus managen, indem der Nutzer beispielsweise Mitarbeitern vor Ort, d.h. in der Nähe der Tiere oder der Herde, Bescheid gibt, wenn eine Handlung erforderlich ist oder ein Tier Unterstützung benötigt, beispielsweise bei der Geburt eins Kalbs oder weil es eine erhöhte Herzfrequenz, eine erhöhte Atemfrequenz oder eine erhöhte Körpertemperatur aufweist.

Die Kennzeichnung eines Tieres erfolgt vorzugsweise durch ein Aufleuchten einer optischen Anzeige in unterschiedlichen Farben, wobei die optische Anzeige vorzugsweise Leuchtmittel umfasst, die beispielsweise von LEDs in verschiedenen Farben gebildet werden. Die Leuchtmittel beziehungsweise die LEDs werden im Sinne der Erfindung bevorzugt als "optische Anzeigemittel" bezeichnet, wobei die optischen Anzeigemittel vorzugsweise an der Erfassungsvorrichtung selbst angeordnet sind. Vorzugsweise sind die optischen Anzeigemittel in einem vorderen Bereich der Erfassungsvorrichtung angeordnet, wobei sich der vordere Bereich der Vorrichtung vorzugsweise zwischen den Nasenöffnungen des Tieres befinden, vorzugsweise außerhalb der Nasenöffnungen. Es kann im Sinne der Erfindung auch bevorzugt sein, dass die optischen Anzeigemittel an dem Halsband und/oder dem Halsring angeordnet vorliegen.

Es ist im Sinne der Erfindung ganz besonders bevorzugt, dass die unterschiedlichen Farben unterschiedliche Bedeutungen haben. Dabei kann es im Sinne der Erfindung bevorzugt sein, dass eine Farbe zu allen Zeiten dieselbe Bedeutung hat. Dies kann beispielsweise die Farbe "rot" sein, die im Sinne der Erfindung signalisiert, dass dringender Handlungsbedarf hinsichtlich eines Tieres besteht. Es ist im Sinne der Erfindung mit anderen Worten bevorzugt, dass hinsichtlich eines Tieres ein dringender Handlungsbedarf besteht, wenn die Erfassungsvorrichtung dieses Tieres, beziehungsweise seine optische Anzeige, ein rotes Licht abgibt. Vorzugsweise kann eine gesonderte Farbe, beispielsweise die Farbe Rot, bei einer starken Abweichung der ermittelten Tierparameter von Vergleichs- und/oder Ausgleichswerten verwendet werden. Solche starken Abweichungen können beispielsweise auch durch ein Blinken der optischen Anzeige angezeigt werden. Dadurch wird dem Nutzer signalisiert, dass mit einem Tier etwas nicht stimmt, wodurch ein besonders schnelles und einfaches Erkennen des betroffenen Tieres im Stall ermöglicht wird. Es ist im Sinne der Erfindung ebenso bevorzugt, dass der Herdenmanager und/oder der Besitzer der Tiere eine Warnmeldung an das mobile Endgerät empfängt. Diese Warnmeldung erfolgt vorzugsweise durch die App. Es kann im Sinne der Erfindung auch bevorzugt sein, dass eine Farbe zu verschiedenen Zeiten unterschiedliche Bedeutungen hat. Der Nutzer des Systems kann eine Farbe zu unterschiedlichen Kennzeichnungszwecken unterschiedliche Bedeutungen zuordnen. Es kann beispielsweise bevorzugt sein, dass zu einem ersten Zeitpunkt, an dem eine Tierherde neu zusammengestellt werden soll, die Farben Grün und Blau verwendet werden, um die Zugehörigkeit eines Tieres zu einer ersten oder einer zweiten Tierherde zu markieren. Es kann aber zu einem anderen, zweiten Zeitpunkt bevorzugt sein, dass eine blaue Farbe beispielsweise angibt, dass ein Tier zur Schlachtung vorgesehen ist, während die Abgabe von grünem Licht angibt, ob ein Tier in der Brunst ist. Indem den einzelnen Farben unterschiedliche Bedeutungen zugeordnet werden können, ist das System beziehungsweise sind die Erfassungsvorrichtungen besonders flexibel und in verschiedenen Zusammenhängen einsetzbar. Dies kommt vorzugsweise dadurch zum Ausdruck, dass die Erfassungsvorrichtungen beziehungsweise das System sowohl zur Erfassung von Tierparametern verwendet werden kann, als auch um einzelne Tiere gegenüber anderen Tieren zu kennzeichnen.

Es kann beispielsweise bevorzugt sein, dass das vorgeschlagene System beziehungsweise das vorgeschlagene Computerprogramprodukt durch die erfassten Tierparameter in der Lage ist, zu bestimmen, dass sich ein Tier in der Brunst befindet. Dieser Zustand des Tieres kann beispielsweise durch die Abgabe von grünem Licht durch die optische Anzeige der Erfassungsvorrichtung angezeigt werden. Wenn das System und/oder das Computerprogramprodukt erkennt/erkennen, dass sich die Anzeichen für die Brunst verstärken, kann beispielsweise weiter grünes Licht mit einer höheren Intensität abgegeben werden oder der Farbton des grünen Lichts kann von einem zunächst hellgrünen zu einem dunkleren Grünton verändert werden. Es ist dabei im Sinne der Erfindung bevorzugt, dass diese Veränderung stufenlos beziehungsweise in Schritten erfolgen kann. Es ist im Sinne der Erfindung darüber hinaus bevorzugt, dass das erstmalige Aufleuchten der grünen LED, die beispielsweise Teil einer optischen Anzeigevorrichtung an der Erfassungsvorrichtung sein kann, als Aktivierung im Sinne der Erfindung bezeichnet wird. Es kann ebenso bevorzugt sein, dass eine Veränderung des Farbtons als Aktivierung im Sinne der Erfindung bezeichnet wird. Durch das Aufleuchten der optischen Anzeige beziehungsweise durch die kontinuierliche Abgabe von Licht eines bestimmten Farbtons kann der Nutzer des Systems beispielsweise das brünstige Tier besonders leicht erkennen und aus der Herde separieren, um es für eine Besamung vorzusehen. Es ist im Sinne der Erfindung bevorzugt, dass die unterschiedlichen Lichtintensitäten beziehungsweise die unterschiedlichen Farbabstufungen unterschiedliche Ausprägungen beziehungsweise Intensitäten eines Zustands, hier der Brunst, angeben können. Die unterschiedlichen Lichtintensitäten beziehungsweise die unterschiedlichen Farbabstufungen zwischen einem hellen und einem dunklen Farbton können im genannten Anwendungsbeispiel vorteilhafterweise dazu führen, dass ein optimaler Besamungszeitraum für ein Tier, zum Beispiel eine Kuh, bestimmt und optimal ausgenutzt wird. Dadurch reduziert sich vorteilhafterweise die Anzahl der erfolglosen Besamungsversuche pro Tier und Zyklus. Darüber hinaus werden unnötige Stressbelastungen für das Tier durch Besamungsversuche zu einem nicht optimalen Besamungszeitpunkt vermieden.

Es kann im Sinne der Erfindung auch bevorzugt sein, dass unterschiedliche Ausprägungen beziehungsweise Intensitäten eines Zustands durch ein Blinken der optischen Anzeige angezeigt werden können. Ein Blinken stellt im Sinne der Erfindung vorzugsweise eine periodisch unterbrochene Abgabe von Licht dar, wobei vorzugsweise unterschiedliche Blinkfrequenzen denkbar sind, beispielsweise um unterschiedliche Ausprägungen beziehungsweise Intensitäten eines Zustands anzuzeigen. Beispielsweise kann der auf Grundlage der erfassten Tierparameter bestimmte Höhepunkt der Brunst durch ein Blinken der optischen Anzeige der Erfassungsvorrichtung angezeigt werden. Den Hinweis darüber, dass der Höhepunkt der Brunst erreicht ist, erhält die Erfassungsvorrichtung beziehungsweise ihre optische Anzeige vorzugsweise durch die PC-Vorrichtung, an der die Auswertung der durch die Erfassungsvorrichtung erfassten Tierparameter erfolgt. Es ist im Sinne der Erfindung bevorzugt, dass die Auswertung auch auf einem mobilen Endgerät oder in der Erfassungsvorrichtung selbst erfolgen kann. Wenn die Auswertung in der Erfassungsvorrichtung selbst erfolgen soll, ist es im Sinne der Erfindung bevorzugt, dass die Erfassungsvorrichtung zu diesem Zwecke einen Mikrokontroller umfasst. Solche Mikrokontroller werden im Sinne der Erfindung vorzugsweise auch als Auswertungseinheiten oder Auswertungsmodule bezeichnet. Für diese Auswertung kann vorzugsweise das vorgeschlagene Computerprogrammprodukt verwendet werden, das vorzugsweise dazu eingerichtet ist, ein Signal mit Informationen über den Zustand des Tieres an die Erfassungsvorrichtung zu übermitteln. Ein besonderer Vorteil der Erfindung besteht darin, dass die Auswertung beziehungsweise eine Überwachung der Tiere hinsichtlich der Tierparameter beziehungsweise der ausgewerteten Daten live erfolgen kann, wobei die Darstellung der Daten vorteilhafterweise besonders übersichtlich ist. Es ist darüber hinaus möglich, dass die Archivierung der Daten ermöglicht wird, so dass der Nutzer einen Überblick über den Verlauf und/oder die Entwicklung der Daten erhalten kann. Wenn die Auswertung im mobilen Endgerät erfolgt, wird das mobile Endgerät vorzugsweise als Auswertungseinheit bezeichnet.

Es kann im Sinne der Erfindung auch bevorzugt sein, dass eine besonders starke ermittelte Abweichung der erfassten Tierparameter von Vergleichs- und/oder Normwerten durch die Abgabe von rotem Licht und/oder ein Blinken durch die optische Anzeige der Erfassungsvorrichtung angezeigt wird. Dadurch kann ein Nutzer des Systems oder der vorgeschlagenen Vorrichtung besonders schnell und wirksam erkennen, wenn ein Tier Besonderheiten aufweist, zum Beispiel, wenn es erkrankt ist, oder wenn in einem Bereich eines Tierstalles unerwünschte äußere Bedingungen, wie zu hohe Temperaturen, herrschen, die zu einer Beeinträchtigung der Gesundheit der dort befindlichen Tiere führt. Beispielsweise kann durch eine solche Anzeige in roter Farbe und/oder durch ein Blinken auf Missstände im Stall aufmerksam gemacht werden, wie zum Beispiel auf einen Herd einer sich ausbreitenden Krankheit, eine ausgefallene Tiertränke oder ein kühler Luftzug durch eine Beschädigung des Stallgebäudes beziehungsweise des Stalldaches.

Vorteilhafterweise bietet die Erfindung somit ein wirksames, tierindividuelles Frühwarnsystem, mit dem zum Beispiel bei starker Abweichung der erhobenen Daten, insbesondere der erfassten Tierparameter, von einer tierindividuellen oder herdenbezogenen Norm, Signale an die Erfassungsvorrichtung und/oder an ein mobiles Endgerät gesendet werden. Wenn die Signale an die Erfassungsvorrichtung gesendet werden, können diese Signale durch eine Empfangseinheit an der Erfassungsvorrichtung erkannt und informationstechnologisch so umgewandelt beziehungsweise verarbeitet werden, dass in Abhängigkeit von den erhaltenen Signalen eine zustandsspezifische Kennzeichnung der einzelnen Tiere einer Herde erfolgen kann. Diese zustandsspezifische Kennzeichnung kann beispielsweise mit einer optischen Anzeige, die insbesondere ein Leuchtmittel umfasst, das seinerseits LEDs umfassen kann, erfolgen. Wenn die Signale an die Erfassungsvorrichtung gesendet werden, können diese Signale an ein mobiles Endgerät gesendet wird, kann es dort beispielsweise in eine Textnachricht, in ein akustisches Signal, eine Vibration oder eine Push-Nachricht umgewandelt werden. Dadurch kann der Nutzer des mobilen Endgeräts darüber informiert werden, wenn mit einem Tier, beispielsweise einer Kuh in einer bestimmten Herde, etwas nicht in Ordnung ist, das Tier Hilfe benötigt oder eine Handlung vorzunehmen ist. Beispielsweise kann die Erfassungsvorrichtung und das vorgeschlagene System auch dazu verwendet werden, dass ein Nutzer darüber informiert wird, wenn eine Kuh kalbt, eine Pferdestute fohlt oder eine Hündin wirft. Dadurch kann die Betreuung der Tiere zu Zeiten, in denen sie Hilfe benötigen, besonders wirksam, aber auch ressourcenschonend optimiert werden. Denn durch die Verwendung der Erfindung kann die Anzahl von "Fehlalarmen" für den Nutzer erheblich reduziert werden und der Nutzer kann wirklich dann zu dem Tier gehen und ihm helfen, wenn eine bestimmte Situation dies erfordert. Dies wird vorteilhafterweise insbesondere dadurch erreicht, dass das vorgeschlagene Computerprogrammprodukt die erfassten Tierparameter besonders genau und mit einer hohen zeitlichen Auflösung auswerten und beispielsweise mit Vergleichs- und/oder Normwerten vergleichen kann. Diese Vergleichs- und/oder Normwerte können beispielsweise tierindividuell oder bezogen auf die Herde des Tieres erhoben oder verwandt werden.

Der Begriff "tierindividuell" bedeutet in Bezug auf die Datenauswertung vorzugsweise, dass die aktuell erfassten Tierparameter mit Vergleichs- und/oder Normwerten verglichen werden, die auf früher erhobene Werte des Tieres beruhen. Es kann beispielsweise im Sinne der Erfindung bevorzugt sein, dass das System über das Sensorpaket der Erfassungsvorrichtung, insbesondere den Druckdifferenzsensor, Druckdifferenzwerte in den Nasenöffnungen des Tieres misst und daraus eine Atemfrequenz ableitet. Diese tierindividuell bestimmte Atemfrequenz kann im System, vorzugsweise auf der PC-Vorrichtung, auf dem mobilen Endgerät oder in der Erfassungsvorrichtung selbst so gespeichert werden, dass die ermittelte tierindividuelle Atemfrequenz später als Vergleichs- und/oder Normwert herangezogen werden kann.

Es kann im Sinne der Erfindung auch bevorzugt sein, dass die Vergleichs- und/oder Normwerte bezogen auf die Herde des Tieres verwendet werden. Dazu kann beispielsweise die Körpertemperatur aller Tiere einer Herde ermittelt werden, wobei ein Temperatursensor vorzugsweise Bestandteil des Sensorpakets der Erfassungsvorrichtung ist. Auf diese Weise kann die Körpertemperatur von jedem einzelnen Tier, vorzugsweise kontinuierlich, d.h. im Dauerbetrieb, ermittelt und vorzugsweise zusammen mit den Daten der anderen Tiere der Herde ausgewertet beziehungsweise verglichen werden. Es kann allerdings auch bevorzugt sein, dass die Vergleichs- und/oder Normwerte in einer Datenbank hinterlegt sind, auf die die Auswertungseinheit oder der Microcontroller der Erfassungsvorrichtung Zugriff haben. Es kann darüber hinaus bevorzugt sein, dass die Körpertemperatur von jedem einzelnen Tier, vorzugsweise nicht kontinuierlich, sondern mit Unterbrechungen, ermittelt und ausgewertet wird, beispielsweise um Akkulaufzeit zu sparen.

Durch die Auswertung der Daten durch die Auswerteeinheit, die beispielsweise Bestandteil des mobilen Endgeräts, einer PC-Vorrichtung oder der Erfassungsvorrichtung ist, kann das vorgeschlagene System vorteilhafterweise Aussagen treffen über akute Stresssituationen eines Tieres, seinem Verhalten, seinem Aufenthaltsort und/oder seinen aktuellen Gesundheitszustand, wobei das System insbesondere dazu eingerichtet ist, die Tiere in Abhängigkeit von den festgestellten Zuständen des Tieres zu kennzeichnen. Diese Kennzeichnung erfolgt bevorzugt mittels einer optischen Anzeige, die in oder an der Erfassungsvorrichtung angeordnet vorliegt. Es ist im Sinne der Erfindung bevorzugt, dass der Begriff "Stress" im Sinne der Erfindung als Krankheit, Verletzung, Brunstgeschehen, Geburtsphase und/oder einer allgemeinen Stresssituation verstanden wird. Eine allgemeine Stresssituation kann zum Beispiel durch eine neue Herdenzusammenstellung, durch die Haltung der Tiere oder durch Schmerzen hervorgerufen werden. Mit dem Begriff "Verhalten" ist im Sinne der Erfindung das Aktivitätsverhalten gemeint beziehungsweise das Verhalten des Tieres in Bezug auf sein Fressen, Trinken, Stehen, Liegen, Laufen und/oder Wiederkäuen beziehungsweise seine Lautäußerungen ("Muhen"). Der aktuelle Gesundheitszustand des Tieres kann vorzugsweise durch Ab- und/oder Vergleich von aktuell ermittelten Tierparameter-Daten mit Vergleichs- und/oder Normwerten ermittelt werden. Dabei liefert die Erfassungsvorrichtung insbesondere Zwischenergebnisse, die vorzugsweise von dem System beziehungsweise in der Auswertungseinheit beziehungsweise durch das Computerprogrammprodukt ausgewertet werden können, wobei diese Auswertung vorteilhafterweise automatisiert und ohne, dass ein Arzt oder Veterinär anwesend sein muss, erfolgt.

Es kann im Sinne der Erfindung auch bevorzugt sein, dass der Nutzer, der beispielsweise Bürotätigkeiten an der PC-Vorrichtung durchführt, das System und das Computerprogrammprodukt für das Management einer Tierherde verwendet. Beispielsweise können mit Hilfe des vorgeschlagenen Systems Signale an die Erfassungsvorrichtung, insbesondere die Nasenringe oder Halsringe, die vorzugsweise von den Tieren der Herde getragen werden, gesendet werden. Wenn beispielsweise eine Tierherde neu zusammengestellt werden soll, kann es im Sinne der Erfindung bevorzugt sein, dass die optischen Anzeigen der Erfassungsvorrichtungen der Tiere, die zu einer ersten Tierherde gehören sollen, beispielsweise ein blaues Licht abgeben, während die optischen Anzeigen der Erfassungsvorrichtungen der Tiere, die zu einer zweiten Tierherde gehören sollen, beispielsweise ein gelbes Licht abgeben. So können neue Teil-Tierherden besonders einfach gebildet werden, ohne mühselig mit den Ohrmarken oder sonstigen Markierungen an den Tieren arbeiten zu müssen. Die Tiere müssen dadurch nicht mit einer neuen, aufwändigen Markierung, wie beispielsweise mit einem Farbstoff, markiert werden, was für die Tiere eine zusätzliche Stressbelastung vermeidet und ihrem Wohlbefinden dient.

Es stellt einen besonderen Vorteil der Erfindung dar, dass die optische Anzeige Licht in verschiedenen Farben abgeben kann, so dass das vorgeschlagene System beziehungsweise die vorgeschlagene Erfassungsvorrichtung besonders flexibel genutzt werden kann. Beispielsweise können die unterschiedlichen Farben zu unterschiedlichen Zeiten unterschiedliche Bedeutungen haben. Es kann beispielsweise bevorzugt sein, dass zu einem Zeitpunkt, an dem eine Tierherde neu zusammengestellt werden soll, die Farben Grün und Blau verwendet werden, um die Zugehörigkeit eines Tieres zu einer ersten oder einer zweiten Tierherde zu markieren. Es kann aber zu einem anderen Zeitpunkt bevorzugt sein, dass eine blaue Farbe beispielsweise angibt, dass ein Tier zur Schlachtung vorgesehen ist, während die Abgabe von grünem Licht angibt, ob ein Tier in der Brunst ist.

Es kann im Sinne der Erfindung beispielsweise eine bestimmte Farbe zur Anzeige durch die optische Anzeige vorgesehen sein, die zur Markierung solcher Tiere dient, die zur Schlachtung vorgesehen sind. Auch hier ist die Vermeidung von Stress für diese Tiere von besonderer Wichtigkeit, da neuere Untersuchungen in den letzten Jahren darauf hindeuten, dass die Ausschüttung von Stresshormonen kurz vor der Schlachtung einen negativen Effekt auf die Qualität des erhaltenen Fleisches hat.

Darüber hinaus ist es unabhängig von der Fleischqualität ein Anliegen der modernen Landwirtschaft, verstärkt auf Bedürfnisse der Tiere und ihr Wohlbefinden zu achten, so dass hier Erfindungen, die die Handhabung von Tierherden und einzelnen Tieren vereinfachen und das Leben für die Tiere stressfreier gestalten, besonders erwünscht sind.

Es ist im Sinne der Erfindung bevorzugt, dass die Erfassungsvorrichtung ein Sensorpaket mit einem oder mehreren zusätzlichen Sensoren zur Erfassung der Tierparameter umfasst. Vorzugsweise umfasst das Sensorpaket einen oder mehrere Sensoren, die ausgewählt sind aus einer Gruppe umfassend Gyrosensor, Accelerometer, Temperatursensor, GPS-Sensor, NFC-Sensor, RFID-Sensor, Luftgütesensor, Sensor für die organisch-chemische Zusammensetzung der Ausatmungsluft dies Tieres und/oder Sensor für die Pulsmessung, wobei auch weitere Sensoren oder beispielsweise zwei oder mehrere Sensoren von einer Sorte in der Erfassungsvorrichtung vorgesehen sein können.

Im Sinne der Erfindung ist der Druckdifferenzsensor dazu eingerichtet ist, eine Druckdifferenz zwischen einem Atmosphärendruck und einem Druck in einem Fluidstrom in mindestens einer Nasenöffnung des Tieres zu messen. Der Druckdifferenzsensor ist vorzugsweise dazu eingerichtet, den Druck in einem Fluidstrom an einem interessierenden Ort, beispielsweise innerhalb einer Nasenöffnung eines Tieres, zu messen. Es ist im Sinne der Erfindung insbesondere bevorzugt, dass der Fluidstrom, vorzugsweise die Ein- und Ausatemluft des Tieres, an einem interessierenden Ort vorbeiströmt. Es ist im Sinne der Erfindung bevorzugt, dass der Atmosphärendruck einen Referenzdruck gegenüber dem Druck im Fluidstrom darstellt, wobei im Sinne der Erfindung auch andere Referenzdrücke als der Atmosphärendruck als Ausgangspunkt für die Bestimmung der Druckdifferenz verwendet werden können. Durch die Verwendung des Druckdifferenzsensors, der am Kopf und/oder am Hals eines Tieres angebracht werden kann, während ein Messfühler des Sensor in mindestens einer Nasenöffnung des Tieres einbringbar ist, wird es vorteilhafterweise ermöglicht, das Tier in seiner natürlichen Bewegung und in Ausübung seiner alltäglichen Verrichtungen, wie zum Beispiel der Nahrungsaufnahme, beim Schlafen, bei der Lautäußerung oder beim Wiederkauen, zu beobachten, ohne erhebliche messungsbedingte Irritationen beim Tier hervorzurufen. Der Begriff "Nahrungsaufnahme" umfasst im Sinne der Erfindung bevorzugt sowohl das Fressen, als auch das Trinken beziehungsweise Saufen. Durch die Messung der Druckdifferenz in der Nase oder in den Nüstern kann das Tier seine üblichen Aktivitäten, wie Fressen, Laufen, Schlafen, Gemolkenwerden, Futteraufnahme und/oder Lautäußerung, weiterhin frei ausüben. Es war vollkommen überraschend, dass mit der Erfindung eine Möglichkeit bereitgestellt werden kann, dass das Tier, bei dem die Tierparameter erfasst werden sollen, durch die Erfindung nicht in seiner Bewegungsfreiheit und seinem natürlichen Verhalten eingeschränkt wird. Es wird davon ausgegangen, dass dem Tier keine Schmerzen oder anderweitige Schäden zugefügt werden oder dass diese Schmerzen oder Schädigungen im Vergleich zu den Schmerzen oder Schädigungen konventioneller Verfahren deutlich reduziert sind. Darüber hinaus ist die Erfindung vorteilhafterweise besonders leicht anzubringen und zu entfernen und es kann tierindividuell angepasst werden, so dass die Beeinträchtigungen für das Tier minimiert werden.

Die Formulierung "Messung einer Druckdifferenz" umfasst im Sinne der Erfindung bevorzugt auch die Messung eines Drucks, denn üblicherweise wird ein Druck dadurch gemessen, dass eine Druckdifferenz zwischen einem ersten Druck und einem zweiten Druck bestimmt wird. Dabei kann es sich bei einem der Drücke um einen Referenzdruck handeln, beispielsweise den Atmosphärendruck. Es ist im Sinne der Erfindung ganz besonders bevorzugt, dass eine Druckdifferenz gemessen wird zwischen einem Atmosphärendruck und einem Druck in einem Fluidstrom an einem interessierenden Ort, wobei es sich bei dem interessierenden Ort vorzugsweise um den Naseninnenraum eines Tieres handelt, durch den bedingt durch das Ein- und Ausatmen des Tieres ein Fluidstrom strömt.

Im Sinne der Erfindung wird der durch den Respirationstrakt eines Tieres erzeugte Ausatmungsdruck nasal mit Hilfe eines Drucksensors ermittelt. Wobei der erzeugte Druck als Differenz zum außenliegenden Atmosphärendruck gemessen wird. Es stellt ein besonderes Verdienst der Erfindung dar, dass die Bestimmung der Atemfrequenz eines Tieres in seinem physiologischen Verhalten ermöglicht wird, wobei vorteilhafterweise, insbesondere durch die Möglichkeit der Langzeitbeobachtung eines Tieres, Veränderungen an dem Tier festgestellt werden können. Es hat sich gezeigt, dass die Atemfrequenz ein wichtiger Indikator für das Wohlbefinden beziehungsweise das Stresslevel eines Tieres darstellt, so dass durch die Kenntnis der Atemfrequenz beziehungsweise seiner Veränderung wichtige Informationen über das beobachtete Tier gewonnen werden können. Diese Informationen können insbesondere dazu verwendet werden, einen Entspannungsgrad oder das Wohlbefinden des Tieres festzustellen und/oder abzuleiten. Eine Auswertung durch (tier)ärztliches Personal ist weder vorgesehen noch erforderlich. Es ist im Sinne der Erfindung insbesondere bevorzugt, dass eine Auswertung der Daten informationstechnisch ohne die Hinzuziehung medizinischen Personals erfolgt. Insbesondere soll das vorliegende Verfahren nicht verwendet werden, um Aussagen über eine konkrete Krankheit zu machen oder eine solche am Tier festzustellen. Eine systematische Benennung von Krankheiten ist im Rahmen des vorgeschlagenen Systems und des vorgeschlagenen Verfahrens nicht vorgesehen. Vorzugsweise liefert die Erfindung einzelne Messwerte oder Datenreihen beziehungsweise Zwischenergebnisse, die bevorzugt lediglich als Grundlage für eine informationstechnologische Auswertung oder Analyse der Daten dienen können, um beispielsweise Rückschlüsse über das Wohlbefinden des Tieres zu ziehen.

Beispielsweise können innere und äußere Faktoren das Wohlbefinden eines Tieres beeinflussen. Innere Faktoren können beispielsweise Krankheiten oder Trächtigkeiten sein, äußere Faktoren können beispielsweise Umverlegungen in einem Stall oder eine Futterumstellung sein. Insofern kann mit der Erfindung ein wichtiger Beitrag zum Wohlbefinden und zur Entspannung des Tieres geleistet werden, wenn Beeinträchtigungen des Tierwohlbefindens einfach und unkompliziert festgestellt und die Ursachen schnell beseitigt werden können. Die Erfindung stellt insofern eine Abkehr vom Stand der Technik dar, als dass die Fachwelt bisher davon ausgegangen war, dass keine Vorrichtungen und Verfahren bereitgestellt werden können, um eine lückenlose Tagesmessung der Tierparameter eines Tieres zu gewährleisten, mit denen beispielsweise der circadiane Rhythmus der Atemfrequenz erfasst werden kann.

Hierbei wird das Fluid, das den Fluidstrom bildet, in dem die Druckdifferenz gemessen wird, von einem Gas gebildet, nämlich der Atemluft, die ein Tier ein- und ausatmet. Es ist im Sinne der Erfindung somit ganz besonders bevorzugt, dass der Fluidstrom von der Atemluft eines Tieres gebildet wird, wobei bekannt ist, dass die Luft, die ein Lebewesen einatmet, üblicherweise eine andere Zusammensetzung aufweist als die Luft, die von dem Lebewesen ausgeatmet wird. Während des Einatmens strömt Luft aus der Umgebung des Kopfes, des Gesichts und/oder der Nase des Tieres durch die Atemwege in die Lunge des Tieres, d.h. es gibt einen Fluidstrom, der von außerhalb des Tierkörpers ins Innere des Tierkörpers strömt. Beim Ausatmen wird "verbrauchte" Luft aus den Lungen des Tieres an die Umgebung des Tieres abgegeben, so dass ein Fluidstrom aus dem Inneren des Tierkörpers an die Außenwelt entsteht. Es ist im Sinne der Erfindung bevorzugt, dass das Einatmen als "Inspiration" und das Ausatmen als "Exspiration" bezeichnet wird. Die unterschiedlichen Richtungen, in denen sich der Fluidstrom bewegt, führen zu geänderten Druckverhältnissen innerhalb der Atemwege des Tieres. Dabei liegen innerhalb des Atemwegs beim Ein- und Ausatmen unterschiedliche absolute Drücke vor. Darüber hinaus ändert sich dadurch auch eine Druckdifferenz zwischen dem Atmosphärendruck und dem Druck in dem Fluidstrom. Es ist im Sinne der Erfindung ganz besonders bevorzugt, dass diese Druckdifferenz zwischen dem Atmosphärendruck und dem Druck in dem Fluidstrom in den Atemwegen eines Tieres gemessen wird, wobei die Druckdifferenz vorzugsweise unterschiedliche Werte annimmt, je nachdem, ob das Tier gerade einatmet oder ausatmet.

Bei dem Fluidstrom handelt es sich um den Strom von Atemgasen in der Nase des Tieres beim Ein- und Ausatmen. Die Erfindung ist darüber hinaus ausgebildet, um in der Ein- und Ausatemluft des Tieres einen Feuchtegehalt zu messen. Dazu umfasst das Sensorpaket der Erfassungsvorrichtung einen Feuchtesensor, sowie einen Sensor zur Erfassung und/oder Auswertung der Atemgase beziehungsweise ihrer Zusammensetzung. Diese Daten können vorteilhafterweise für die Ketosefrüherkennung verwendet werden.

Eine Übermittlung von Daten zwischen dem Drucksensor und der Auswertungseinheit kann vorzugsweise drahtlos oder drahtbehaftet erfolgen, wobei insbesondere eine Übertragung der Daten mittels einer Internetverbindung und/oder einem WLAN bevorzugt ist. Es ist im Sinne der Erfindung bevorzugt, dass insbesondere eine

Übertragung der Daten mittels Wireless, vorzugsweise WLAN, Wi-Fi, Bluetooth, NFC und/oder einer Internetverbindung vorgesehen ist. Die NFC-Übertragung kann insbesondere zur Nahübertragung an einem Roboter vorgesehen sein. Durch eine solche drahtlose Datenübertragung wird der Verkabelungsaufwand wesentlich reduziert und die Anwendung des Systems in Verbindung mit einem Tier deutlich vereinfacht. Insbesondere kann das System, wenn es an dem Tier befestigt vorliegt, unbeaufsichtigt verwendet werden, weil keine Drähte oder Kabel vorhanden sind, in denen sich das Tier einwickeln oder durch die sich das Tier schädigen kann. Insbesondere wird dadurch eine Langzeitüberwachung des Tieres ermöglicht, weil eine Beaufsichtigung durch einen Operator des Systems nicht erforderlich ist. Durch die Übermittlung der Daten, insbesondere der Zwischenergebnisse, wird darüber hinaus vorteilhafterweise ermöglicht, dass die Auswertung der Daten an einem anderen Ort erfolgt. Somit kann die Auswertung der Daten vorteilhafterweise ortsunabhängig erfolgen. Die Auswertung der Daten muss somit nicht in der Nähe des Tieres, beispielsweise in seinem Stall, erfolgen, sondern die Daten können an einen Ort übertragen werden, der besser für die Auswertung der Daten beziehungsweise die Aufstellung einer Auswertungseinheit geeignet ist, als ein Kuhstall. Ein Stall stellt üblicherweise eine große Halle dar, in der eine Vielzahl von Tieren gehalten wird.

Es ist im Sinne der Erfindung bevorzugt, dass die Gyrosensoren und Accelerometer dazu eingerichtet sind, Winkel- und/oder Geschwindigkeitsveränderung beim Tier zu erfassen. Die mit den Gyrosensoren und Accelerometern ermittelten Daten Dienen zur Auswertung der Kopfbewegung, Fressverhalten und/oder dem körperlichen Einfluss eines anderen Tiers, ohne darauf beschränkt zu sein. Ferner können dadurch Lahmheiten und/oder das Verhalten in Bezug auf das Fressen, Liegen, Schlafen, Stehen, Laufen, allg. Aktivität, Schritte am Tag und/oder das Kopfschlafen ermittelt und ausgewertet werden.

Vorzugsweise umfasst das vorgeschlagene System ferner eine PC-Vorrichtung, wobei die PC-Vorrichtung dazu eingerichtet ist, die erfassten Tierparameter auszuwerten. Die Auswertung durch die PC-Vorrichtung umfasst insbesondere die Ermittlung einer Atemfrequenz des Tieres aus den ermittelten Tierparametern, insbesondere den Druckdifferenzwerten. Die erfassten Tierparameter stellen im Sinne der Erfindung vorzugsweise Zwischenergebnisse dar, die informationstechnologisch weiterverarbeitet und/oder ausgewertet werden können.

Es ist insbesondere bevorzugt, dass auf der PC-Vorrichtung, dem mobilen Endgerät und/oder der Erfassungsvorrichtung selbst ein Computerprogrammprodukt zur Auswertung und/oder Analyse der erfassten Tierparameter vorliegen kann. Der Begriff "Computerprogrammprodukt" beschreibt im Sinne der Erfindung bevorzugt eine Software oder ein Computerprogramm, das dazu eingerichtet ist, Daten zu verarbeiten und auszuwerten. Es ist im Sinne der Erfindung besonders bevorzugt, dass eine graphische Auswertung der erfassten Tierparameter erfolgt, wobei der graphischen Auswertung vorzugsweise eine graphische Auftragung der Daten zugrunde liegt. Es ist im Sinne der Erfindung bevorzugt, dass die Erfassungsvorrichtung, die Auswertungseinheit, das mobile Endgerät und/oder die PC-Vorrichtung Mittel zum Speichern der Daten in den unterschiedlichen Auswertungsschritten umfassen.

Eine graphische Auftragung der Druckdifferenz gegenüber einer Zeitachse führt vorzugsweise zu einer periodischen Wiederholung von im Wesentlichen ähnlichen Vorgängen, wobei eine Atemperiode durch einen Anstieg der Druckdifferenz beim Ausatmen und einem Absinken der Druckdifferenz beim Einatmen gekennzeichnet ist. Inspiration und Exspiration werden in einer solchen Auftragung vorzugsweise von einem Exspirationspeak voneinander getrennt, wobei zwischen zwei Exspirationspeaks üblicherweise ein Minimum der Druckdifferenz vorliegt. Indem die ermittelte Druckdifferenz mittels einer Auswertungseinheit ausgewertet wird, kann eine Atemfrequenz des Tieres ermittelt werden. Es ist beispielsweise möglich, die Anzahl der Exspirationspeaks pro Zeiteinheit zu bestimmen, wobei die Anzahl von Atemvorgängen pro Zeiteinheit im Sinne der Erfindung als Atemfrequenz bezeichnet wird. Die Atemfrequenz wird vorzugsweise in der Einheit "1/s" oder "Hertz" angegeben. Es kann im Sinne der Erfindung auch bevorzugt sein, die Zeiteinheit "Minute", "Stunde" oder "Tag" als Grundlage der Atemfrequenz zu verwenden, ohne darauf beschränkt zu sein. Der Begriff "Atemvorgang" bezeichnet im Sinne der Erfindung bevorzugt einen vollständigen Atemzyklus des Tieres, also einen vollständigen Einatmungsvorgang und einen vollständigen Ausatmungsvorgang. Wenn ein Tier also beispielsweise 35 Mal pro Minute ein- und ausatmet, beträgt die entsprechende Atemfrequenz 35 min⁻¹. In der graphischen Auftragung äußert sich eine solche Atemfrequenz beispielsweise durch das Vorhandensein von 35 Exspirationspeaks pro Minute.

Der GPS-Sensor (GPS = *Global Positioning System*) ermöglicht es vorteilhafterweise, dass das System in der Lage ist, Aussagen zum konkreten Aufenthaltsort des Tieres zu machen. Die Erfassungsvorrichtung kann dazu mit einem GPS-Sender ausgestattet sein, wobei das System, die Auswertungseinheit und/oder das Computerprogrammprodukt vorzugsweise dazu eingerichtet sind, die vom GPS-Sensor erhaltenen Daten auszuwerten und daraus den Aufenthaltsort eines Tieres vorzugsweise tierindividuell zu ermitteln.

Im Sinne der Erfindung ist bevorzugt der Aufbau bzw. die Nutzung eines Wireless-Systems zur Positionierung der Tiere vorgesehen. Dies bedeutet im Sinne der Erfindung bevorzugt, dass beispielsweise mindestes drei Sender und//oder Empfänger an beispielsweise drei Positionen mit gleichem Abstand positioniert werden, wobei die drei Standorte der Sensoren vorzugsweise ein im Wesentlichen gleichseitiges Dreieck bilden. Die Erfinder haben erkannt, dass es mit dieser bevorzugten Anordnung unter Ausnutzung der Empfangsstärke möglich ist, den Abstand des Sensors zu ermitteln und die Position des Tieres bestimmen. Diese Anwendung ist besonders für den Einsatz in einem Stall geeignet, da Ställe häufig gut abgeschirmt sind und ein GPS-Signal dort Fehler erzeugen könnte.

Es kann im Sinne der Erfindung vorgesehen sein, dass die PC-Vorrichtung und/oder das mobile Endgerät eine Anzeigenvorrichtung umfassen, wobei auf diesen Anzeigenvorrichtungen Karten und/oder Pläne, beispielsweise von dem Stall, in dem sich das Tier aufhält, oder von dem Gelände, auf dem sich das Tier bewegt, angezeigt werden. Es ist im Sinne der Erfindung ganz besonders bevorzugt, dass der Aufenthaltsort des Tieres auf diesen Karten und/oder Plänen durch das System angezeigt beziehungsweise markiert werden kann. Durch die Verwendung und Auswertung der GPS-Daten kann ein Auffinden eines Tieres, das zu einem bestimmten Zweck gesucht wird, wesentlich erleichtert werden. Dies auch insbesondere deshalb, weil ein gesuchtes Tier mit dem System beziehungsweise durch die optische Anzeige der Erfassungsvorrichtung, die es trägt, markiert beziehungsweise gekennzeichnet werden kann. Dazu kann beispielsweise eine Farbe verwendet werden, die gerade nicht zur Anzeige anderer zustandsspezifischer Kennzeichnungen verwendet wird. Es ist im Sinne der Erfindung mit anderen Worten bevorzugt, dass ein gesuchtes Tier mittels der optischen Anzeige gekennzeichnet werden kann, wobei die Kennzeichnung insbesondere da-durch erfolgt, dass die optische Anzeige blinkt oder Licht einer bestimmten zuvor festgelegten Farbe aussendet.

Es ist im Sinne der Erfindung bevorzugt, dass die Vorrichtung einen RFID-Chip zur Erkennung der Tiere umfasst (*RFID* = *radio frequency identification*). Vorzugsweise kann zur Erkennung und/oder Identifizierung der Tiere auch ein NFC-Sensor oder ein NFC-Chip verwendet werden. Es ist im Sinne der Erfindung ganz besonders bevorzugt, dass der RFID-Chip zur Identifizierung der Tiere verwendet werden kann. Mit diesem RFID-Chip können zum Beispiel einzelne Tiere erkannt werden, wenn sie an bestimmten Stellen des Stalles vorbeigehen, zum Beispiel wenn sie sich zum Melkstand, zur Tränke oder zu den Futtertrögen begeben. Die so erhaltenen Identifizierungsdaten können vorteilhafterweise mit den erfassten Tierparametern, die vorzugsweise mit den Sensoren des Sensorpakets der Erfassungsvorrichtung ermittelt werden, zusammengebracht und bei der Auswertung der Daten gemeinsam berücksichtigt werden. Dazu werden die Identifizierungsdaten vorzugsweise ebenfalls an die Auswertungseinheit des Systems übermittelt, wobei auch die Übertragung dieser Daten vorzugsweise drahtlos erfolgt.

Durch die gemeinsame Auswertung der Daten des Sensorpakets und des RFID- und/oder NFC-Chips ergeben sich synergistische Effekte bei der Beurteilung des Zustands eines Tieres, die deutlich über das zu erwartende Maß hinausgehen. RFID- und/oder NFC-Chip oder-sensoren bilden vorzugsweise Mittel zur Bestimmung der Tierposition bzw. zur Bestimmung des Aufenthalts eines Tieres oder mehrerer Tiere. Die Mittel zur Bestimmung der Tierposition können vorzugsweise auch zur Erkennung und/oder Identifizierung von Tieren verwendet werden. Beispielsweise können die Daten und Zwischenergebnisse dazu verwendet werden, dass das System von sich aus, d.h. vorzugsweise automatisch, vorschlägt, dass eine Erkrankung eines Tieres vorliegt. Wenn beispielsweise ein Tier über einen längeren Zeitraum vermeidet, zum Melkstand zu gehen und darüber hinaus eine erhöhte Körpertemperatur aufweist, kann die Auswerteeinheit von sich aus vorschlagen, dass das betroffene Tier gegebenenfalls an einer Euterentzündung leidet. Die Anzahl der Melkstandbesuche wird dabei vorzugsweise mit Hilfe des RFID-Chips ermittelt, während die Körpertemperatur des Tieres mit dem Temperatursensor des Sensorpaktes der Erfassungsvorrichtung ermittelt wird. Es ist im Sinne der Erfindung bevorzugt, dass mit der Erfindung ein solcher automatisch ermittelter Krankheitsverdacht als Signal an die Erfassungsvorrichtung übermittelt werden kann, wobei die Erfassungsvorrichtung vorzugsweise unter Verwendung der optischen Anzeige dazu eingerichtet ist, das Tier zu kennzeichnen, indem eine rote LED an der Vorrichtung aktiviert wird, was beispielsweise dazu führen kann, dass die Vorrichtung rotes Licht abgibt oder blinkt. Es ist im Sinne der Erfindung ganz besonders bevorzugt, dass die Ermittlung eines Krankheitsverdachts durch die Erfindung automatisch beziehungsweise automatisiert erfolgen kann, wobei hierfür insbesondere kein Arzt oder kein geschultes medizinisches Personal vorhanden sein muss.

Es ist im Sinne der Erfindung bevorzugt, dass die Erfassungsvorrichtung als Nasenring ausgebildet ist. Es ist insbesondere bevorzugt, dass die Vorrichtung ringförmig ausgebildet ist und in Nasenöffnungen eines Tieres eingeführt werden kann. Es ist im Sinne der Erfindung insbesondere bevorzugt, dass der Ring an einer Stelle unterbrochen ist und auf diese Weise zwei im Wesentlichen symmetrische Hälften bildet, wobei je eine Hälfte des Nasenrings in eine Nasenöffnung des Tieres eingeführt werden kann. Dabei liegt die Unterbrechungsstelle im Bereich der Nasenscheidewand des Tieres vor beziehungsweise die Nasenscheidewand liegt im Bereich der Unterbrechungsstelle des Nasenrings vor. Es ist im Sinne der Erfindung insbesondere bevorzugt, dass die Befestigung der Vorrichtung über die knorpeligen Endstücke innerhalb der Tiernase beziehungsweise der Nasenscheidewand des Tieres erreicht wird, wobei die Erfinder erkannt haben, dass dadurch eine besonders effektive, unkomplizierte und störungsfreie Befestigung der Vorrichtung ermöglicht wird. Es ist im Sinne der Erfindung bevorzugt, dass die Endstellen der beiden Hälften des Nasenrings Haltebacken umfassen, die im Anwendungsfall des Nasenrings ebenfalls in den Nasenöffnungen des Tieres vorliegen. Vorzugsweise drücken die Haltebacken dergestalt gegen die Nasenscheidewand des Tieres, dass der Nasenring dadurch wirksam im Bereich der Nase des Tieres befestigt wird. Vorzugsweise ist der Druck aber auch so gering, dass das Tier durch das Tragen des Rings nicht wesentlich bei seinen täglichen Verrichtungen gestört wird. Es ist im Sinne der Erfindung bevorzugt, dass die Erfassungsvorrichtung beziehungsweise der Nasenring ein elastisches Material umfasst beziehungsweise aus einem solchen gebildet wird. Es ist im Sinne der Erfindung bevorzugt, dass es durch die Elastizität des Materials der Vorrichtung ermöglicht wird, die beiden Hälften der Ringkonstruktion im Bereich der Unterbrechungsstelle leicht auseinanderzudrücken, um den Nasenring besonders einfach und für das Tier schonend in die Nasenöffnungen des Tieres einzuführen. Es ist im Sinne der Erfindung bevorzugt, dass die Tierparameter direkt über den Nasenring ermittelt werden können, indem der Nasenring ein Sensorpaket umfassend mindestens einen der oben genannten Sensoren umfasst.

Es ist im Sinne der Erfindung bevorzugt, dass die Vorrichtung eine Ringkonstruktion umfasst, wobei die Ringkonstruktion zwei im Wesentlichen symmetrische Hälften aufweist, zwischen denen an einer Stelle die Ringkonstruktion unterbrochen ist, wobei an den Endstellen die beiden Hälften der Ringkonstruktion jeweils Haltebacken vorgesehen sind, die die Vorrichtung durch Druck gegen eine Nasenscheidewand des Tieres in der Nase des Tieres befestigen.

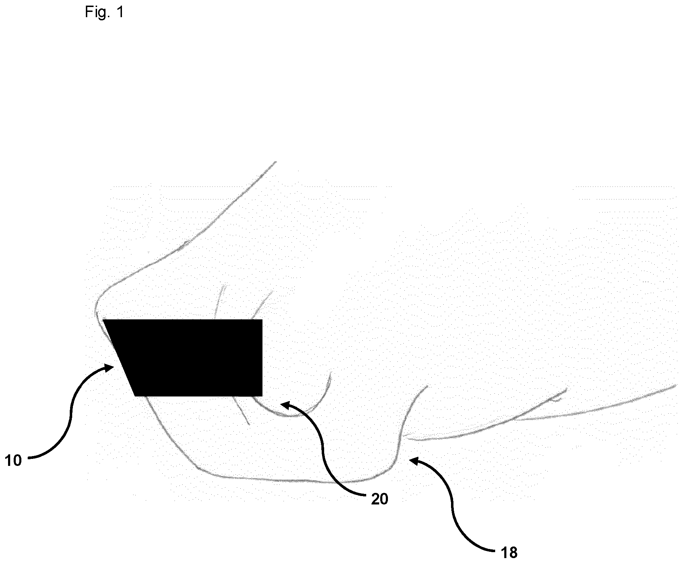

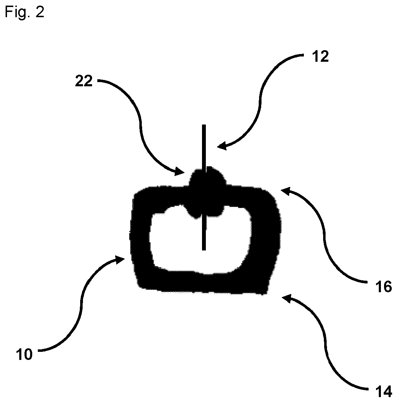

Es ist im Sinne der Erfindung darüber hinaus bevorzugt, dass die Vorrichtung beispielsweise bei Kühen, insbesondere Rindern, Pferden oder Hunden verwendet wird, wobei die Vorrichtung auch bei anderen Tieren, deren Nasenöffnungen für eine solche Befestigung geeignet sind, verwendet werden kann. Vorzugsweise werden die Nasenöffnungen bei einigen dieser Tiere auch als "Nüstern" bezeichnet. Vorzugsweise umfasst die Vorrichtung eine ringförmige Konstruktion, wobei der Begriff "ringförmig" im Sinne der Erfindung bevorzugt nicht als geometrisch perfekter Kreis verstanden werden soll, sondern als im Wesentlichen elliptischer Ring, der beispielsweise auch Ecken und Kanten umfassen kann. Es kann im Sinne der Erfindung auch bevorzugt sein, dass ein Befestigungspunkt der Vorrichtung von dem weichen Nasenaußenteilen, den knorpeligen Gebilden innerhalb der Nase und/oder der Nasenscheidewand gebildet wird. Insbesondere erfolgt die Befestigung der Vorrichtung somit über die Einbeziehung dieser Körperteile des Tieres. Eine mögliche dreidimensionale Ausprägung einer solchen Ringkonstruktion ist in Figur 2 dargestellt. Es ist im Sinne der Erfindung insbesondere bevorzugt, dass der Ring zwei längere und zwei kürzere Seiten aufweist, die einander im Wesentlichen gegenüberliegen. Es ist im Sinne der Erfindung bevorzugt, dass die eine längere Seite die Vorderseite des Naserings darstellt, die von außen an dem Tier sichtbar ist. Dies bedeutet im Sinne der Erfindung bevorzugt, dass diese eine längere Seite des Nasenrings im Gesicht des Tieres, insbesondere zwischen den Nasenöffnungen des Tieres und von außen sichtbar angeordnet vorliegt. Dieser sichtbare Bereich der Vorrichtung ist beispielsweise in Figur 1 schematisch als schwarzer Balken dargestellt. Es ist im Sinne der Erfindung bevorzugt, dass dieser sichtbare Bereich des Nasenrings einer der beiden länglichen Seiten der Vorrichtung entspricht. Vorzugsweise liegt im vorderen Bereich der Erfassungsvorrichtung die optische Anzeige zur Kennzeichnung der Tiere vor.

Es ist im Sinne der Erfindung bevorzugt, dass die zwei kürzeren Seiten des Nasenrings von dem sichtbaren Bereich des Nasenrings abgehen und in die Nasenöffnungen des Tieres führen. Es ist im Sinne der Erfindung bevorzugt, dass sich an die kürzeren Seiten der Vorrichtung Ecken und/oder Biegungen anschließen. Es ist im Sinne der Erfindung bevorzugt, dass der Ring nicht vollständig geschlossen ist, sondern dass der Ring an einer Stelle eine Öffnung aufweist. Die Öffnung des Nasenrings liegt vorzugsweise im Wesentlichen in der Mitte der zweiten längeren Seite des Nasenrings vor, wobei diese zweite längere Seite bevorzugt als Hinterseite der Erfassungsvorrichtung bezeichnet wird. Es ist im Sinne der Erfindung bevorzugt, dass die beiden im Wesentlichen symmetrisch ausgebildeten Hälften des Nasenrings an der Unterbrechungsstelle aufeinanderstoßen können, wobei die Endbereiche der Nasenringhälften vorzugsweise als Endstellen bezeichnet werden. Es ist im Sinne der Erfindung ganz besonders bevorzugt, dass die Vorrichtung beziehungsweise die Ringkonstruktion aus einem elastischen Material gebildet wird, so dass die beiden Hälften des Nasenrings vorsichtig und unter Aufbringung eines gewissen Drucks auseinander gedrückt beziehungsweise gespreizt werden können. Es ist im Sinne der Erfindung bevorzugt, dass durch das Auseinanderdrücken der Ringhälften eine geöffnete Stellung des Rings erreicht werden kann, wobei der Ring in dieser geöffneten Stellung besonders einfach an dem Tier angebracht werden kann. Dazu drückt ein Nutzer die Hälften der Ringkonstruktion auseinander und führt die beiden Hälften in je eine Nasenöffnung des Tieres ein. Wenn die eine längere Seite des Rings, die die Vorderseite der Vorrichtung bildet, den Zwischenbereich des Tieres zwischen seiner Nasenöffnung berührt, lässt der Nutzer die beiden Endstellen der Ringhälften langsam und vorsichtig aufeinander zu bewegen, so dass die beiden Haltebacken des Rings auf je einer Seite der Nasenscheidewand des Tieres anliegen. Vorzugsweise liegen die Haltebacken auf gleicher Höhe innerhalb der Nase des Tieres vor und werden lediglich durch die Nasenscheidewand des Tieres voneinander getrennt. Dieser Zustand wird im Sinne der Erfindung bevorzugt als geschlossener Zustand der Vorrichtung beziehungsweise des Nasenrings bezeichnet.

Die Vorrichtung umfasst vorzugsweise ein elastisches Material oder die Vorrichtung ist ganz oder teilweise aus einem elastischen Material gebildet. Es ist im Sinne der Erfindung bevorzugt, dass das elastische Material der Vorrichtung das Auseinanderdrücken beziehungsweise Spreizen des Rings in eine geöffnete Stelle erlaubt, ohne dass das Material beziehungsweise der daraus gefertigte Ring früher oder später bricht. Es ist im Sinne der Erfindung darüber hinaus bevorzugt, dass das elastische Material so ausgewählt wird, dass die Haltebacken im geschlossenen Zustand auf der einen Seite einen sicheren Halt der Haltebacken beziehungsweise der Vorrichtung in der Nase beziehungsweise in den Nasenöffnungen des Tieres ermöglichen und auf der anderen Seite nicht zu fest auf die Nasenscheidewand des Tieres drücken, da das Tier dies gegebenenfalls als unangenehm oder schmerzhaft empfinden könnte. Solche für das Tier unangenehmen Empfindungen durch den Nasenring werden durch die Konstruktion, Beschaffenheit und/oder Materialauswahl der Vorrichtung vorteilhafterweise vermieden, was im Kontext der vorliegenden Erfindung besonders wichtig ist, da anderenfalls fehlerhafte Tierparameter erhoben würden, die durch das Tragen des Nasenrings verfälscht sind.

Es ist im Sinne der Erfindung bevorzugt, dass die Vorrichtung Kunststoff und/oder Metall umfasst oder aus diesen Materialien gebildet ist. Beide Materialien erfüllen die hohen Anforderungen an Sterilität und Hygiene, die bei der Verwendung im Bereich der Tierhaltung zu erfüllen sind, insbesondere wenn die Tiere zur Nahrungsmittelproduktion gehalten werden. Die Verwendung von Kunststoff ermöglicht eine zügige und kostengünstige Herstellung der Ringkonstruktion, die vorzugsweise die Struktur der Vorrichtung vorgibt. Insbesondere können Nasenringe aus Kunststoff in Massenproduktionsverfahren hergestellt werden, wobei es im Sinne der Erfindung bevorzugt ist, dass eine Nachbearbeitung der erzeugten Kunststoffrohlinge erfolgt, um beispielsweise unerwünschte Grate und scharfe Kanten zu entfernen. Die Verwendung von Kunststoff ist auch deswegen bevorzugt, weil Kunststoff ein schlechter Wärmeleiter ist und sich das Material so für das Tier nicht kalt anfühlt. Die Verwendung von Metall kann aufgrund der Langlebigkeit des Materials bevorzugt sein. Darüber hinaus kann Metall auf relativ hohe Temperaturen erhitzt werden, um beispielsweise sterilisiert zu werden. Dies kann vorteilhaft sein, wenn ein Nasenring nach einer Anwendung an einem ersten Tier für ein anderes, zweites Tier genutzt werden soll, wobei zwischen der ersten und der zweiten Anwendung eine Sterilisation und/oder eine Reinigung vorgesehen ist.

Es ist im Sinne der Erfindung bevorzugt, dass die Endstellen der Ringhälften von Haltebacken gebildet werden, wobei die Haltebacken vorzugsweise als Verdickungen im Endbereich der Ringhälften ausgebildet sind. Die Haltebacken können in ihrer Form an in-ear-Lautsprecher erinnern, wobei die Haltebacken insbesondere im Wesentliche glatte und eben ausgebildete Vorderseiten aufweisen, die, wenn die Vorrichtung am Tier befestigt ist, an den beiden Seiten der Nasenscheidewand des Tieres anliegen. Es ist im Sinne der Erfindung bevorzugt, dass die Haltebacken an diesen Vorderseiten eine größere Grundfläche aufweisen, als die Ringkonstruktion im hinteren Bereich der Vorrichtung. Durch die vergrößerten Grundflächen verteilt sich der Druck besonders gleichmäßig auf einen größeren Bereich der Nasenscheidewand des Tieres, so dass sich das Tier möglichst wenig durch das Tragen der Vorrichtung gestört fühlt. Der hintere Bereich des Nasenrings ist im Sinne der Erfindung derjenige Bereich, der die Unterbrechungsstelle aufweist und der vorzugsweise von einer der beiden längeren Seiten der Ringkonstruktion gebildet wird. Es ist im Sinne der Erfindung insbesondere bevorzugt, dass der hintere Teil der Vorrichtung derjenige Teil der Vorrichtung ist, der im Anwendungsfall in den Nasenöffnungen des Tieres vorliegt und insbesondere von außen nicht sichtbar ist.

Es kann im Sinne der Erfindung auch bevorzugt sein, dass die Vorrichtung als Halsring ausgebildet ist. Es kann in dieser Ausführungsform der Erfindung bevorzugt sein, dass die Vorrichtung selber als Halsring ausgebildet ist, wobei der Begriff "Halsring" im Sinne der Erfindung bevorzugt bedeutet, dass eine Vorrichtung im Wesentlichen ringförmig ausgebildet ist und um den Hals eines Tieres gelegt werden kann. Es kann im Sinne der Erfindung ebenso bevorzugt sein, dass die Vorrichtung eine Vorrichtung zur Befestigung eines Sensorpakets am Hals eines Tieres umfasst, so dass die Vorrichtung mit der Befestigungsvorrichtung an dem Hals des Tieres angebracht werden kann. Die Befestigungsvorrichtung kann beispielsweise als Gurt oder Halfter ausgebildet sein, wobei die Befestigungsvorrichtung beispielsweise aus einem Fasermaterial, Kunststoff, Naturmaterialien und/oder Leder gebildet sein kann beziehungsweise diese Materialien umfassen kann.

In einem weiteren Aspekt betrifft die Erfindung ein System zur Erfassung und Auswertung von Tierparametern, wobei das System mindestens eine Erfassungsvorrichtung und eine PC-Vorrichtung umfasst. Das System ist dadurch gekennzeichnet, dass die von der mindestens einen Vorrichtung erfassten Tierparameter an die PC-Vorrichtung zur Auswertung übermittelt werden, wobei die PC-Vorrichtung dazu eingerichtet ist, in Abhängigkeit von den Auswertungsergebnissen Signale an die Vorrichtung zur Erfassung von Tierparametern zu übermitteln, so dass eine optische Anzeige der Vorrichtung in Abhängigkeit von den übermittelten Signalen aktiviert wird. Die technischen Wirkungen, Vorteile und Definitionen, die für die Erfassungsvorrichtung beschrieben werden, gelten analog für das vorgeschlagene System und umgekehrt.

Das vorgeschlagene System kann zusätzlich ein mobiles Endgerät umfassen, wobei auf dem mobilen Endgerät eine App installiert ist. Die App kann vorzugsweise Bedienoberflächen umfassen, auf denen unterschiedliche Informationen und Daten vorzugsweise in aufbereiteter Form dargestellt werden können. Beispielsweise können die Informationen von dem Computerprogrammprodukt und/oder der Auswertungseinheit so ausgewertet und/oder aufbereitet werden, dass pro Seite beziehungsweise pro Bedienoberfläche die Daten für je ein Tier angezeigt werden. Es kann aber auch bevorzugt sein, dass Übersichtsansichten vorgesehen sind oder solche Anzeigen, die die statistische Auswertung einzelner Tiere, Teilmengen von Tieren der Herde oder der Gesamtherde betreffen. Vorzugsweise können die Daten auch graphisch aufbereitet sein und/oder als Grafiken angezeigt werden. Dazu umfasst das mobile Endgerät vorzugsweise eine Anzeigenvorrichtung, wobei auf der Anzeigenvorrichtung die erfassten Tierparameter oder Auswertungen, die auf den erfassten Tierparameter beruhen, angezeigt werden können. Insbesondere ist die Darstellung von Live-Daten möglich, die insbesondere für eine Langzeitüberwachung eines Tieres nützlich ist. Es ist im Sinne der Erfindung bevorzugt, dass die Anzeigenvorrichtung des mobilen Endgeräts als Berührbildschirm ausgebildet ist. Vorzugsweise können auch Tablet-PCs, Palms, PDA-Geräte, Handheld-Geräte und andere mobile Kommunikationsvorrichtungen als mobile Endgeräte im Sinne der Erfindung genutzt werden.

Das vorgeschlagene System kann darüber hinaus eine Dashboard-Vorrichtung beziehungsweise eine Dashboard-Anwendung umfassen. Ein Dashboard ist im Sinne der vorliegenden Erfindung bevorzugt ein Software-Programmprodukt mit Mitteln zur Visualisierung von Informationen. Die vorgeschlagene Dashboard-Anwendung umfasst beispielsweise eine grafische Benutzeroberfläche, um die erfassten Tierparameter oder die auf den erfassten Tierparametern basierenden Auswertungsdaten benutzerdefiniert in Übersichten, Grafiken, Diagrammen und/oder weiteren Zusammenstellungsformen darzustellen. Eine Dashboard-Vorrichtung kann beispielsweise ähnlich wie ein Tablet-PC ausgebildet sein und in einem Stall installiert sein, so dass der Nutzer vor Ort Informationen über die Tierherde oder einzelne Tiere abrufen oder einsehen kann. Es ist im Sinne der Erfindung bevorzugt, dass die Dashboard-Vorrichtung dazu eingerichtet ist, dass eine Dashboard-Anwendung auf der Dashboard-Vorrichtung betrieben werden kann.

Die Dashboard-Vorrichtung und die Erfassungsvorrichtung, die PC-Vorrichtung und/oder das mobile Endgerät umfassen jeweils Mittel zum Aufbau einer Informationsaustausch-Verbindung, mit der die Informationen zwischen den genannten Vorrichtungen ausgetauscht werden können. Somit kann ein Nutzer der Erfindung vorteilhafterweise auf die Informationen, die von der Erfassungsvorrichtung erfasst beziehungsweise von der Auswertungseinheit ausgewertet werden, zugreifen, ohne dass er sich in räumlicher Nähe des Tierstalls aufhalten muss. Auf der Dashboard-Vorrichtung können vorteilhafterweise Informationen von mehreren Tierherden gleichzeitig dargestellt werden. Ein überraschender Vorteil der Dashboard-Vorrichtung besteht darin, dass der Anwender die ermittelten Daten selbst in der gewünschten Darstellungsform anpassen kann, ohne dazu über Programmierkenntnisse und -fähigkeiten zu verfügen. Dadurch wird eine hohe Benutzerfreundlichkeit der Dashboard-Vorrichtung sowie des vorgeschlagenen Systems gewährleistet. Insbesondere wird dadurch, dass keine Programmierkenntnisse seitens des Anwenders erforderlich sind, die Hemmschwelle zur Benutzung der Dashboard-Vorrichtung und zur Anwendung des Dashboards erheblich herabgesetzt.

Vorzugsweise umfasst die Erfassungsvorrichtung eine Energiequelle, vorzugsweise eine Batterie und/oder einen Akku. Der Begriff "Akku" wird im Sinne der Erfindung synonym zu den Begriffen "Akku-Vorrichtung" oder "Akkumulator" verwendet. Es ist im Sinne der Erfindung ganz besonders bevorzugt, dass die Erfassungsvorrichtung einen aufladbaren Akku umfasst, der vorzugsweise dazu eingerichtet ist, seine Energie aus der Körperwärme des Tieres zu gewinnen. Dazu verfügt der Akku über mindestens ein Peltier-Element, mit dem Wärme in elektrische Energie umwandelbar ist. Die Verwendung eines auf diese Weise aufladbaren Akkus ist besonders vorteilhaft, weil sich dadurch eine mögliche Tragedauer der Erfassungsvorrichtung an dem Tier deutlich verlängert und die Erfassungsvorrichtung weniger häufig ausgetauscht werden muss, wobei jeder Austauschvorgang eine zusätzliche Stressbelastung für das Tier darstellt. Außerdem kann durch die Verwendung der aufladbaren Akkus auf die Vorsehung anderer Energiequellen verzichtet werden, so dass die Erfassungsvorrichtung besonders ressourcenschonend arbeitet.

Es kann im Sinne der Erfindung auch eine Akkulösung ohne Peltier-Element vorgesehen sein. Darüber hinaus ist, vorzugsweise im sonnenreichen Sommer, eine zusätzliche Zuführung von Solarenergie durch Aufladung einer Solarzelle denkbar.

In einem weiteren Aspekt betrifft die Erfindung ein Computerprogrammprodukt zum Management einer Tierherde. Das Computerprogrammprodukt ist dadurch gekennzeichnet, dass es, wenn es auf der PC-Vorrichtung, der Vorrichtung zur individuellen Erfassung von Tierparametern und/oder dem mobilen Endgerät installiert vorliegt, die folgenden Schritte vornimmt:
a) Auswertung von Tierparametern, die mit dem Sensorpaket erfasst werden, wobei das Sensorpaket einen oder mehrere Sensoren umfasst,
b) Bestimmung eines Zustands des Tieres,

In einer weiteren bevorzugten Ausführungsform der der Erfindung kann das Computerprogrammprodukt die folgenden weiteren Schritte ausführen:
c) Übermittlung eines Signals mit Informationen über den Zustand des Tieres an die Vorrichtung zur individuellen Erfassung von Tierparametern, wobei durch das übermittelte Signal eine optische Anzeige an der Vorrichtung zur individuellen Erfassung von Tierparametern aktiviert wird,
d) Anzeige des Zustandes des Tieres durch die optische Anzeige.

Vorzugsweise kann die Vorrichtung beziehungsweise das System auch zur Tiererkennung und/oder zur Tieridentifizierung verwendet werden. Wenn der Herdenmanager im Stall oder auf dem Außengelände ein bestimmtes Tier sucht, beispielsweise Kuh 796, so kann er an seinem PC oder auf seinem mobilen Endgerät - vorzugsweise über die App - eingeben, dass er dieses Tier sucht. Das System und die Vorrichtung sind vorzugsweise dazu eingerichtet, dass dann nur die optischen Anzeigemittel der gesuchten Kuh 796 aufleuchten oder dass diese in einer bestimmten "Such-Farbe" aufleuchten. Es kann im Sinne der Erfindung auch bevorzugt sein, dass die optischen Anzeigemittel der gesuchten Kuh 796 beispielsweise zu blinken beginnen, wenn die Kuh 796 gesucht wird. Auf diese Weise kann eine Kuh besonders einfach, schnell und für das gesuchte Tier schonend gesucht und gefunden werden.

Es ist im Sinne der Erfindung bevorzugt, dass die Auswertung der von der Erfassungsvorrichtung beziehungsweise von den Sensoren des Sensorpakets erfassten Tierparameter insbesondere in einem Abgleich der erfassten Tierparameter mit Vergleichs- und/oder Normwerten besteht oder in einer Umrechnung von ermittelten Werten in Auswertungswerte, so dass die ermittelten Tierparameter beispielsweise mit Vergleichs- und/oder Normwerten verglichen werden können. Es ist im Sinne der Erfindung insbesondere bevorzugt, dass durch die Auswertung der Tierparameter der Zustand eines Tieres unter verschiedenen Aspekten festgestellt und/oder bestimmt werden kann. Insbesondere wird es durch die Auswertung der Tierparameter möglich, die Ausprägungen einzelner Aspekte, Verhaltensweisen oder Zustände des Tieres zu ermitteln. Die Bestimmung des Zustands eines Tieres gemäß des oben genannten Schrittes b) kann beispielsweise die Feststellung von Abweichungen der erfassten Tierparameter von Vergleichs- und/oder Normwerten umfassen. Die Auswertung kann ferner die Feststellung von Ausprägungen von Zuständen des Tieres umfassen. Die Aktivierung der optischen Anzeige bewirkt vorzugsweise, dass sich die optische Anzeige der Erfassungsvorrichtung ändert. Dies kann beispielsweise dadurch erfolgen, dass eine LED, die zuvor nicht geleuchtet hat, zu Leuchten beginnt. Eine Aktivierung der optischen Anzeige kann beispielsweise auch zu einer Farbänderung des abgegebenen Lichts führen, beispielsweise dadurch, dass anstatt eines grünen Lichts dann blaues Licht abgegeben wird. Dies kann zum Beispiel dadurch erreicht werden, dass eine Steuerung oder Steuervorrichtung innerhalb der Erfassungsvorrichtung dazu eingerichtet ist, eine zum Beispiel blaue LED, die vorzugsweise blaues Licht abgibt, auszuschalten und eine grüne LED, die vorzugsweise grünes Licht abgibt einzuschalten, oder umgekehrt. Die Aktivierung der optischen Anzeige kann sich auch dadurch äußern, dass die optische Anzeige zu Blinken beginnt oder damit aufhört.

Es ist im Sinne der Erfindung ganz besonders bevorzugt, dass es sich bei der optischen Anzeige zum Beispiel um ein Leuchtmittel aufweisend eine oder mehrere *light emitting diodes* (LEDs) handeln kann, wobei die LEDs vorzugsweise in unterschiedlichen Farben leuchten können. Vorzugsweise umfasst die Erfassungsvorrichtung ein Leuchtmittel zur Kennzeichnung der unterschiedlichen Zustände des Tieres. Es ist im Sinne der Erfindung bevorzugt, dass die optische Anzeige im Wesentlichen im Dauerbetrieb arbeitet und dass sich beispielsweise die Farbe des abgegebenen Lichts ändert, wenn der Nasen- oder Halsring ein Signal erhält. Ein solches Signal führt im Sinne der Erfindung vorzugsweise zur Aktivierung der optischen Anzeige der Erfassungsvorrichtung. Es stellt einen wesentlichen Vorteil der Erfindung dar, dass mit der Erfindung unterschiedliche Zustände des Tieres mit einer zustandsspezifischen Kennzeichnung gekennzeichnet werden können. Dazu wird vorzugsweise die Erfassungsvorrichtung mit ihrer optischen Anzeige verwendet. Es ist im Sinne der Erfindung insbesondere bevorzugt, dass unterschiedliche Ausprägungen von Zuständen des Tieres mit der zustandsspezifischen Kennzeichnung markiert werden können.

In einer weiteren bevorzugten Ausführungsform kann das Computerprogramm den folgenden weiteren Schritt ausführen:
e) Übermittlung eines Signals mit Informationen über den Zustand des Tieres an ein mobiles Endgerät, wobei das mobile Endgerät eine Anzeigenvorrichtung umfasst, wobei auf der Anzeigenvorrichtung die erfassten Tierparameter oder Auswertungen, die auf den erfassten Tierparameter beruhen, anzeigbar sind.

Es ist mit anderen Worten bevorzugt, dass ein Signal mit Informationen über den Zustand des Tieres an ein mobiles Endgerät übermittelt werden kann, wobei das mobile Endgerät eine Anzeigenvorrichtung umfasst, wobei auf der Anzeigenvorrichtung die erfassten Tierparameter oder Auswertungen, die auf den erfassten Tierparameter beruhen, angezeigt werden können.

Es wird darüber hinaus ein Verfahren zum Herdenmanagement offenbart, jedoch nicht beansprucht, das die genannten Schritte a) bis d) beziehungsweise die genannten Schritte a) bis e) umfasst. Die Definitionen, technischen Wirkungen und Vorteile, die hinsichtlich der Erfassungsvorrichtung und des Systems beschrieben wurden, gelten analog für das Verfahren.

Es ist im Sinne der Erfindung bevorzugt, dass der Nutzer Tiere, die eine Vorrichtung tragen, vorzugsweise manuell über das mobile Endgerät kennzeichnet. Mit anderen Worten ist die Vorrichtung dazu eingerichtet, dass der Nutzer Tiere, die eine Vorrichtung tragen, über das mobile Endgerät kennzeichnen kann. Mithin betrifft die Erfindung auch ein System zur Kennzeichnung von Tieren, wobei das System mindestens eine vorgeschlagene Vorrichtung und einen PC und/oder ein mobiles Endgerät umfasst. Das System ist dadurch gekennzeichnet, dass der Nutzer Kennzeichnungsinformationen an dem PC und/oder an dem mobilen Endgerät eingibt, die mittels einer Kommunikationsverbindung an die mindestens eine Vorrichtung übermittelt werden. Die Erfassungsvorrichtung ist vorzugsweise dazu eingerichtet, mittels der optischen Anzeigemittel ein optisches Signal in Abhängigkeit von der übermittelten Kennzeichnungsinformation abzugeben. Bei dem optischen Signal kann es sich beispielsweise um ein Leuchten der optischen Anzeigemittel in einem bestimmten Farbton handeln oder um ein Blinken der Erfassungsvorrichtung.

Mit anderen Worten ist es im Sinne der Erfindung bevorzugt, dass ein System zur individuellen Kennzeichnung von Tieren mindestens eine vorgeschlagene Erfassungsvorrichtung, sowie eine PC-Vorrichtung und/oder ein mobiles Endgerät umfasst, wobei einzelne Tiere an der PC-Vorrichtung und/oder an dem mobilen Endgerät ausgewählt werden können, wobei die PC-Vorrichtung und/oder das mobile Endgerät dazu eingerichtet ist/sind, Signale hinsichtlich der ausgewählten Tiere an die Vorrichtung(en) zu übermitteln, so dass eine optische Anzeige der Vorrichtung in Abhängigkeit von den übermittelten Signalen aktiviert wird. Es ist im Sinne der Erfindung insbesondere bevorzugt, dass eine optische Anzeige der Vorrichtung in Abhängigkeit von ausgewählten Tieren aktiviert wird. Es ist in diesem Zusammenhang besonders bevorzugt, dass insbesondere diejenigen optischen Anzeigen aktiviert werden, die von den ausgewählten Tieren getragen werden. Es kann allerdings auch bevorzugt sein, dass sowohl die Vorrichtungen der ausgewählten Tiere aktiviert werden, als auch die Vorrichtungen der nicht-ausgewählten Tiere. Gemäß der ersten Alternative kann es bevorzugt sein, dass Tiere zur Besamung ausgewählt werden und dass die optischen Anzeigen der Erfassungsvorrichtung dieser zur Besamung vorgesehen Tiere aufleuchten oder zu blinken beginnen. Gemäß der zweiten Alternative sollen beispielsweise die Tiere gekennzeichnet werden, die zur Schlachtung vorgesehen sind, wobei die zur Schlachtung vorgesehenen Tiere mit einer grün leuchtenden Erfassungsvorrichtung markiert werden, während die nicht zur Schlachtung vorgesehen Tiere mit einer gelb leuchtenden optischen Anzeige der Erfassungsvorrichtung markiert werden.

Die Kennzeichnungsfunktion soll an folgendem Beispiel verdeutlicht werden: Wenn beispielsweise der Besamer in Abwesenheit des Landwirts einen landwirtschaftlichen Betrieb aufsucht, auf dem eine Herde mit Rindern lebt, von denen einige Tiere von dem Besamer besamt werden sollen, kann der Nutzer, d.h. der Landwirt oder ein Mitarbeiter auf dem landwirtschaftlichen Betrieb, die zu besamenden Tiere auf die oben beschriebe Weise kennzeichnen, und zwar vorzugsweise über sein mobiles Endgerät oder vom Arbeits-PC des Landwirts aus. Beispielsweise können die zu besamenden Tiere in der Farbe blau gekennzeichnet werden. Der Besamer sieht die blauen Tiere und weiß, dass er diese blau gekennzeichneten Tiere besamen muss. Mit anderen Worten erkennt der Besamer die zu besamenden Tiere an ihrer - beispielsweise - blauen Kennzeichnung und kann die Besamung dann unkompliziert anhand der Kennzeichnung vornehmen.

Bevorzugt ist, dass das mobile Endgerät eine Anzeigenvorrichtung umfasst, wobei auf der Anzeigenvorrichtung die erfassten Tierparameter oder Auswertungen, die auf den erfassten Tierparametern beruhen, anzeigbar sind.

Bevorzugt ist außerdem, dass eine Auswertung der erfassten Tierparameter in einer Auswerteeinheit der PC-Vorrichtung erfolgt.

Aus den weiteren Unteransprüchen und der nachfolgenden Beschreibung sind weitere Vorteile, Merkmale und Einzelheiten der Erfindung zu entnehmen. Dort erwähnte Merkmale können jeweils einzeln für sich oder auch in beliebiger Kombination erfindungswesentlich sein. So kann auf die Offenbarung zu den einzelnen Erfindungsaspekten stets wechselseitig Bezug genommen werden. Die Zeichnungen dienen lediglich beispielhaft der Klarstellung der Erfindung und haben keinen einschränkenden Charakter.

Die Erfindung wird durch die nachfolgende Figur näher beschrieben; es zeigt:
- Figur 1: Darstellung einer bevorzugten Erfassungsvorrichtung, die an einem Tier angebracht vorliegt
- Figur 2: Darstellung einer bevorzugten Ausführungsform der Erfassungsvorrichtung als Nasenring
- Figur 3: Darstellung einer bevorzugten Ausführungsform der Erfassungsvorrichtung für ein Pferd
- Figur 4: Darstellung einer bevorzugten Ausführungsform der Erfassungsvorrichtung mit einer Spange
- Figur 5: Darstellung einer bevorzugten Ausführungsform der Erfassungsvorrichtung, die als Stent ausgebildet ist
- Figur 6: Darstellung einer bevorzugten Ausführungsform der Erfassungsvorrichtung, die als Stent ausgebildet ist

Figur 1 zeigt eine bevorzugte Ausführungsform der Erfassungsvorrichtung (10), die in den Nasenöffnungen (20) eines Tieres (18) vorliegt. Insbesondere zeigt Figur 1 die Vorderseite (14) der Erfassungsvorrichtung (10). Die Erfassungsvorrichtung (10) umfasst vorzugsweise ein Sensorpaket zur Erfassung von Tierparametern und eine optische Anzeige zur Kennzeichnung von Tieren und zur Anzeige der Zustände eines Tieres (18) in Bezug auf verschiedene Gesundheits- und Wohlbefindensaspekte des Tieres (18). Die Erfassungsvorrichtung (10) kann vorzugsweise auch die allgemeine Tierkennzeichnung umfassen, die vorzugsweise durch eine Kennnummer angegeben wird. Darüber hinaus kann die Erfassungsvorrichtung (10) einen RFID-Chip umfassen, mit dem das Tier (18) identifiziert werden kann.

Vorzugsweise können mit der Erfassungsvorrichtung (10) die folgenden Daten tierindividuell erhoben werden: Stress, Verhalten, Gesundheitsparameter, Aufenthaltsort (mittels GPS-Erkennung). Es ist ganz besonders bevorzugt, dass die Erfassungsvorrichtung (10) mit Sensoren ausgestattet ist, die gemeinsam ein Sensorpaket bilden.

Figur 2 zeigt die Darstellung einer bevorzugten Ausführungsform der Erfassungsvorrichtung (10) als Nasenring. Es ist im Sinne der Erfindung bevorzugt, dass die Erfassungsvorrichtung (10) zwei im Wesentlichen symmetrische Hälften umfasst, wobei die Nasenscheidewand (12) als Symmetrieachse fungieren kann. Die Erfassungsvorrichtung (10) umfasst vorzugsweise Haltebacken (22), wobei je einer der beiden Haltebacken (22) an je einer Seite der Nasenscheidewand (12) des Tieres (18) anliegt. Vorteilhafterweise wird der Nasenring (10) dadurch innerhalb der Nasenöffnungen (20) des Tieres (18) befestigt. Es ist im Sinne der Erfindung bevorzugt, dass die Seite der Erfassungsvorrichtung (10), die die Haltebacken (22) und die Unterbrechungsstelle aufweist, als Rückseite (16) der Erfassungsvorrichtung (10) bezeichnet wird.

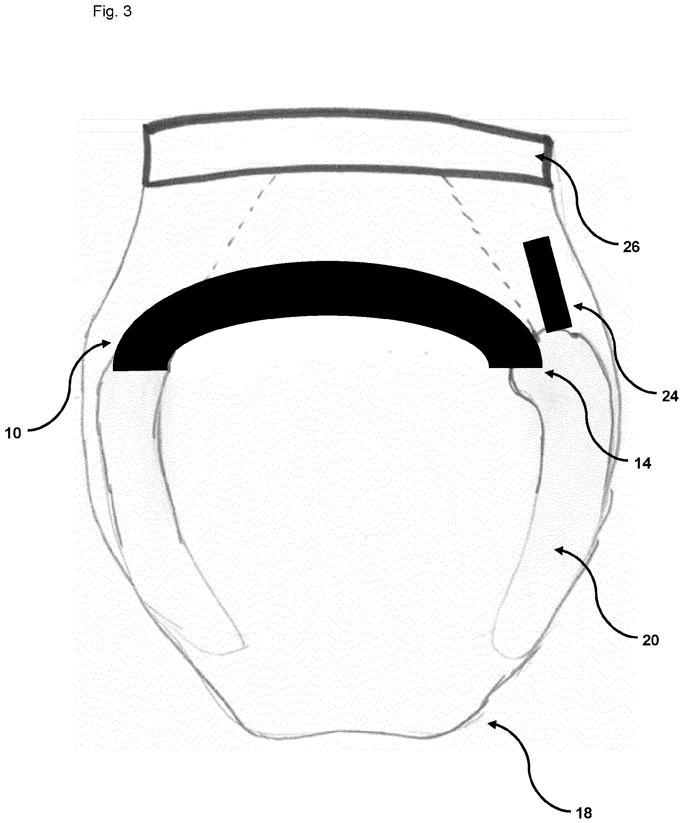

Figur 3 zeigt eine bevorzugte Befestigungsmöglichkeit der vorgeschlagenen Vorrichtung (10) an einem Tier (18), insbesondere an einem Pferd. Vorzugsweise kann die Vorrichtung (10), insbesondere ihre Vorderseite (14), auf den Bereich des Tierkopfes aufgelegt werden, der sich zwischen den Nasenöffnungen (20) des Tieres (18) befindet. Es kann im Sinne der Erfindung bevorzugt sein, dass Teile der Vorrichtung (10) in die Nasenöffnungen (20) des Tieres (18) hineinragen, um eine Befestigungswirkung zu erzielen. Zusätzlich kann es im Sinne der Erfindung bevorzugt sein, dass die Vorrichtung (10) einen Clip oder eine Spange (24) umfasst, mit dem/der die Vorrichtung (10) im Nasenbereich des Tieres (18) befestigt werden kann. Der Clip oder die Spange (24) sind beispielhafte Ausführungsformen von Befestigungsmitteln, die vorzugsweise dazu eingerichtet sind, die Vorrichtung (10) im Bereich der Nasenöffnungen (20) des Tieres zu befestigen.

Die in Figur 3 dargestellte Ausführungsform der Erfindung führt vor allem bei Pferden dazu, dass die Tiere (18) die Vorrichtung (10) nicht abstreifen können. Darüber hinaus sind die clip- oder spangenartig ausgebildeten Befestigungsmittel (24) besonders einfach und kostengünstig herzustellen, beispielsweise auch in Massenproduktionsverfahren. Somit wird mit dieser Ausführungsform der Erfindung eine besonders einfach und unaufwändige Befestigungsmöglichkeit der Vorrichtung (10) bereitgestellt, die darüber hinaus kostengünstig hergestellt werden kann. Im oberen Bereich von Figur 3 ist ein Halfter (26) abgebildet, dass üblicherweise von Pferden getragen wird.

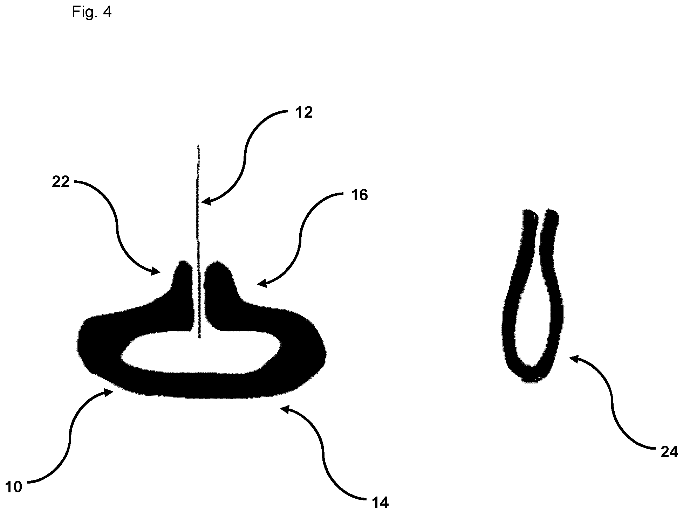

Figur 4 zeigt eine bevorzugte Ausführungsform der vorgeschlagenen Erfassungsvorrichtung (10) (links im Bild) und eine bevorzugte Ausführungsform einer Spange (24) (rechts im Bild). Die in Figur 4 dargestellte bevorzugte Ausführungsform der Vorrichtung (10) weist auch Haltebacken (22) auf, die mit der Nasenscheidewand (12) des Tieres (18) oder den knorpeligen Endstücken im Naseninnenraum des Tieres (18) so zusammenwirken, dass die Vorrichtung (10) sicher und für das Tier (18) im Wesentlichen unlösbar, schmerzfrei und unmerklich in den Nasenöffnungen (20) des Tieres (18) befestigt wird. Figur 4 zeigt auch die Lage von Vorderseite (14) und Rückseite (16) der Vorrichtung (10) in Bezug auf den Nasenbereich des Tieres (18).

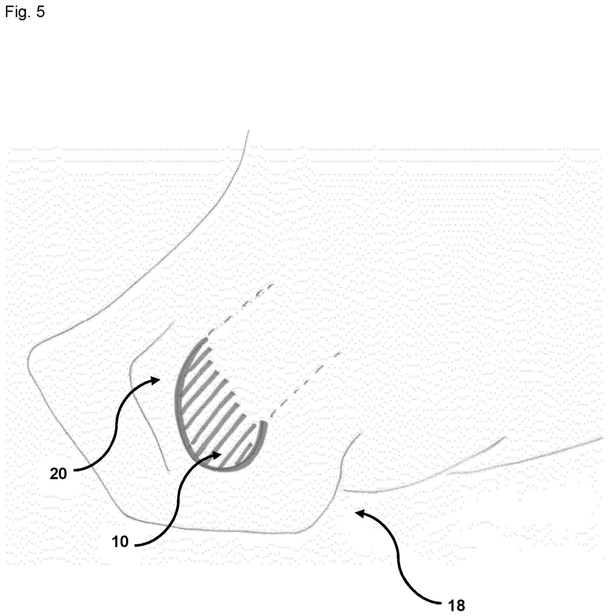

Figur 5 zeigt eine bevorzugte Ausführungsform der Erfindung, die als Stent ausgebildet ist. Bei einer stentartigen Ausführung der Vorrichtung (10) wird die Vorrichtung (10) in einem zusammengefalteten Zustand in eine oder beide Nasenöffnungen (20) des Tieres (18) eingeführt, beispielsweise indem der Stent bzw. die Vorrichtung (10) in die Nasenöffnungen (20) hineingeschoben wird, so dass die Vorrichtung (10) im Naseninnenraum des Tieres (18) zu liegen kommt. Vorteilhafterweise kann sich die Vorrichtung (10) in ihrer stentartigen Ausprägung im Naseninnenraum des Tieres (18) auseinanderfalten. Mit anderen Worten ist es im Sinne der Erfindung bevorzugt, dass die Vorrichtung (10) in dieser Ausführungsform der Erfindung dazu eingerichtet ist, sich im Naseninnenraum eines Tieres (18) zu entfalten, so dass sie dann in einem auseinandergefalteten oder entfalteten Zustand vorliegt. Vorteilhafterweise passt sich die Vorrichtung (10) beim Entfalten - vorzugsweise automatisch - den räumlichen und/oder anatomischen Gegebenheiten des Naseninnenraumes des Tieres (18) an, so dass es diesen besonders gut und enganliegend auskleidet. Dadurch wird das Tier (18), vorzugsweise das Pferd, möglichst wenig in seinem normalen Leben gestört.

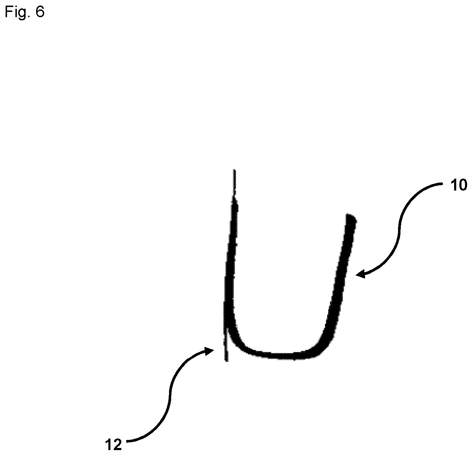

Figur 6 zeigt eine bevorzugte Ausführungsform einer stentartig ausgebildeten Ausführungsform der Vorrichtung (10) im entfalteten Zustand in der Nase des Tieres (18). Insbesondere zeigt Figur 6, wie die Vorrichtung (10) in dieser Ausführungsform der Erfindung gegenüber der Nasenscheidewand (12) des Tieres (18) angeordnet ist.

### Bezugszeichenliste:

- 10: Erfassungsvorrichtung, Nasenring
- 12: Nasenscheidewand
- 14: Vorderseite der Erfassungsvorrichtung
- 16: Rückseite der Erfassungsvorrichtung
- 18: Tier
- 20: Nasenöffnung des Tieres, Nüstern
- 22: Haltebacken
- 24: Spange oder Clip
- 26: Halfter

## Patentansprüche

1. Vorrichtung (10) zur individuellen Erfassung von Tierparametern, wobei die Vorrichtung (10) ein Sensorpaket mit einem oder mehreren Sensoren zur Erfassung der Tierparameter umfasst, wobei die Vorrichtung (10) am Kopf und/oder am Hals eines Tieres (18) anbringbar ist, wobei das Sensorpaket einen Druckdifferenzsensor umfasst und ein Messfühler des Druckdifferenzsensor in mindestens einer Nasenöffnung des Tieres anbringbar ist, wobei der Druckdifferenzsensor vorzugsweise dazu eingerichtet ist, einen relativen Druck in einem Fluidstrom von Atemgasen in der Nase des Tieres zu messen,
**dadurch gekennzeichnet, dass**
das Sensorpaket weiterhin einen Sensor für die Messung eines Feuchtegehalts in dem Fluidstrom umfasst und wobei aus den erfassten Tierparametern Rückschlüsse auf einen Zustand des Tieres (18) ableitbar sind und/oder unterschiedliche Zustände des Tieres (18) mit einer zustandsspezifischen Kennzeichnung an der Vorrichtung (10) anzeigbar sind.

2. Vorrichtung (10) nach Anspruch 1
**dadurch gekennzeichnet, dass**
das Sensorpaket einen oder mehrere Sensoren umfasst, die ausgewählt sind aus einer Gruppe umfassend Gyrosensor, Accelerometer, Temperatursensor, GPS-Sensor, NFC-Sensor, RFID-Sensor, Luftgütesensor, Sensor für die organisch-chemische Zusammensetzung der Ausatmungsluft dies Tieres und/oder Sensor für die Pulsmessung.

3. Vorrichtung (10) nach Anspruch 1 oder 2
**dadurch gekennzeichnet, dass**
die Vorrichtung (10) einen RFID-Chip zur Erkennung der Tiere (18) umfasst.

4. Vorrichtung (10) nach einem oder mehreren der vorhergehenden Ansprüche
**dadurch gekennzeichnet, dass**
die Vorrichtung (10) einen NFC-Sensor zur Erkennung der Tiere (18) umfasst.

5. Vorrichtung (10) nach einem oder mehreren der vorhergehenden Ansprüche
**dadurch gekennzeichnet, dass**
die Vorrichtung (10) als Nasenring ausgebildet ist.

6. Vorrichtung (10) nach Anspruch 5
**dadurch gekennzeichnet, dass**
die Vorrichtung (10) eine Ringkonstruktion umfasst, wobei die Ringkonstruktion zwei im Wesentlichen symmetrische Hälften aufweist, zwischen denen an einer Stelle die Ringkonstruktion unterbrochen ist, wobei an den Endstellen die beiden Hälften der Ringkonstruktion jeweils Haltebacken (22) vorgesehen sind, die die Vorrichtung (10) durch Druck gegen eine Nasenscheidewand (12) des Tieres (18) in der Nase des Tieres (18) befestigen.

7. Vorrichtung (10) nach einem oder mehreren der Ansprüche 1 bis 4
**dadurch gekennzeichnet, dass**
die Vorrichtung (10) als Halsband ausgebildet ist.

8. Vorrichtung (10) nach einem oder mehreren der Ansprüche 1 bis 4
**dadurch gekennzeichnet, dass**
die Vorrichtung (10) als Stent ausgebildet ist oder einen Stent umfasst.

9. Vorrichtung (10) nach einem oder mehreren der vorhergehenden Ansprüche
**dadurch gekennzeichnet, dass**
die Vorrichtung (10) ein Befestigungsmittel (24) umfasst, wobei das Befestigungsmittel (24) als Spange oder Clip ausgebildet ist.

10. Vorrichtung (10) nach einem oder mehreren der vorhergehenden Ansprüche
**dadurch gekennzeichnet, dass**
die Vorrichtung (10) mit den Befestigungsmitteln (24) an einem Nasenflügel und/oder Nüstern des Tieres (18) anbringbar ist.

11. Vorrichtung (10) nach einem oder mehreren der vorhergehenden Ansprüche
**dadurch gekennzeichnet, dass**
die Vorrichtung (10) ein Leuchtmittel zur Kennzeichnung der unterschiedlichen Zustände des Tieres (18) umfasst.

12. Vorrichtung (10) nach einem oder mehreren der vorhergehenden Ansprüche
**dadurch gekennzeichnet, dass**
eine Auswertung der erfassten Tierparameter in der Vorrichtung (10) selbst erfolgt.

13. System zur Erfassung und Auswertung von Tierparametern, wobei das System mindestens eine Vorrichtung (10) nach einem oder mehreren der vorgehenden Ansprüche umfasst, sowie eine PC-Vorrichtung
**dadurch gekennzeichnet, dass**
die von der mindestens einen Vorrichtung (10) erfassten Tierparameter an die PC-Vorrichtung zur Auswertung übermittelt werden, wobei die PC-Vorrichtung dazu eingerichtet ist, in Abhängigkeit von den Auswertungsergebnissen Signale an die Vorrichtung zur Erfassung von Tierparametern zu übermitteln, so dass eine optische Anzeige der Vorrichtung in Abhängigkeit von den übermittelten Signalen aktiviert wird.

14. System zur individuellen Kennzeichnung von Tieren, wobei das System mindestens eine Vorrichtung (10) nach einem oder mehreren der Ansprüche 1 bis 12 umfasst, sowie eine PC-Vorrichtung und/oder ein mobiles Endgerät
**dadurch gekennzeichnet, dass**
einzelne Tiere an der PC-Vorrichtung und/oder an dem mobilen Endgerät ausgewählt werden können, wobei die PC-Vorrichtung und/oder das mobile Endgerät dazu eingerichtet ist/sind, Signale hinsichtlich der ausgewählten Tiere an die Vorrichtung(en) (10) zu übermitteln, so dass eine optische Anzeige der Vorrichtung (10) in Abhängigkeit von den übermittelten Signalen aktiviert wird.

15. Computerprogrammprodukt zum Management einer Tierherde
**dadurch gekennzeichnet, dass**
es, wenn es auf einer Vorrichtung (10) zur individuellen Erfassung von Tierparametern nach einem oder mehreren der vorhergehenden Ansprüchen 1 - 12 installiert vorliegt, die folgenden Schritte vornimmt:
a) Auswertung von Tierparametern, die mit dem Sensorpaket erfasst werden, wobei das Sensorpaket einen oder mehrere Sensoren umfasst,
b) Bestimmung eines Zustand des Tieres (18), und vorzugsweise auch die folgenden Schritte:
c) Übermittlung eines Signals mit Informationen über den Zustand des Tieres an die Vorrichtung zur individuellen Erfassung von Tierparametern, wobei durch das übermittelte Signal eine optische Anzeige an der Vorrichtung (10) aktiviert wird,
d) Anzeige des Zustandes des Tieres (18) durch die optische Anzeige, und vorzugsweise auch den folgenden zusätzlichen Schritt:
e) Übermittlung eines Signals mit Informationen über den Zustand des Tieres (18) an ein mobiles Endgerät, wobei das mobile Endgerät eine Anzeigenvorrichtung umfasst, wobei auf der Anzeigenvorrichtung die erfassten Tierparameter oder Auswertungen, die auf den erfassten Tierparametern beruhen, anzeigbar sind.

## Claims

1. A device (10) for individually detecting animal parameters
wherein
the device (10) comprises a sensor package with one or more sensors for the detection of animal parameters, wherein the device (10) is attachable to the head and/or neck of an animal (18), wherein the sensor package comprises a pressure difference sensor and a sensor of the pressure difference sensor is attachable in at least one nostril of the animal, wherein the pressure difference sensor is preferably adapted to measure a relative pressure in a fluid flow of respiratory gases in the nose of the animal,
**characterized in that**
the sensor package further comprises a sensor for measuring a moisture content in the fluid flow and
wherein conclusions about a condition of the animal (18) can be derived from the detected animal parameters and/or different conditions of the animal (18) can be displayed with a condition-specific marking on the device (10).

2. The device (10) of claim 1
**characterized in that**
the sensor package comprises one or more sensors selected from a group comprising gyro sensor, accelerometer, temperature sensor, GPS sensor, NFC sensor, RFID sensor, air quality sensor, sensor for the organic-chemical composition of the exhaled air of the animal and/or sensor for pulse measurement.

3. The device (10) of claim 1 or 2
**characterized in that**
the device (10) comprises an RFID chip for identifying the animals (18).

4. The device (10) of any one or more of the preceding claims
**characterized in that**
the device (10) comprises an NFC sensor for recognising the animals (18).

5. The device (10) of any one or more of the preceding claims
**characterized in that**
the device (10) is configured as a nose ring.

6. The device (10) of claim 5
**characterized in that**
the device (10) comprises a ring structure, wherein the ring structure has two substantially symmetrical halves between which the ring structure is interrupted at one location, wherein retaining jaws (22) are provided respectively at the end locations of the two halves of the ring structure, which fasten the device (10) in the nose of the animal (18) by pressure against a nasal septum (12) of the animal (18).

7. The device (10) of any one or more of claims 1 to 4
**characterized in that**
the device (10) is configured as a collar.

8. The device (10) of any one or more of claims 1 to 4
**characterized in that**
the device (10) is configured as a stent or comprises a stent.

9. The device (10) of any one or more of the preceding claims
**characterized in that**
the device (10) comprises a fastening means (24), wherein the fastening means (24) is configured as a clasp or clip.

10. The device (10) of any one or more of the preceding claims
**characterized in that**
the device (10) is attachable to a nostril and/or nostrils of the animal (18) by the fastening means (24).

11. The device (10) of any one or more of the preceding claims
**characterized in that**
the device (10) comprises a light means for indicating the different conditions of the animal (18).

12. The device (10) of any one or more of the preceding claims
**characterized in that**
an evaluation of the detected animal parameters is performed in the device (10) itself.

13. A system for detecting and evaluating animal parameters, wherein the system comprises at least one device (10) of any one or more of the preceding claims, and a PC device
**characterized in that**
the animal parameters detected by the at least one device (10) are transmitted to the PC device for evaluation, wherein the PC device is configured to transmit signals to the device for detecting animal parameters based on the evaluation results, so that a visual display of the device is activated based on the transmitted signals.

14. A system for individually identifying animals, wherein the system comprises at least one device (10) of any one or more of claims 1 to 12, and a PC device and/or a mobile terminal
**characterized in that**
individual animals can be selected at the PC device and/or at the mobile terminal, wherein the PC device and/or the mobile terminal is/are configured to transmit signals regarding the selected animals to the device(s) (10) so that a visual display of the device (10) is activated in response to the transmitted signals.

15. A computer program product for managing an animal herd
**characterized in that**
when installed on a device (10) for individually detecting animal parameters of any one or more of the preceding claims 1 - 12, the computer program product performs the following steps:
a) evaluating animal parameters sensed by the sensor package, wherein the sensor package comprises one or more sensors,
b) determining a condition of the animal (18),
and preferably also the following steps:
c) transmitting a signal containing information about the condition of the animal to the device for individually detecting animal parameters, wherein the transmitted signal activates a visual display on the device (10),
d) display of the condition of the animal (18) by the optical display,
and preferably also the following additional step:
e) transmitting a signal with information about the condition of the animal (18) to a mobile terminal, wherein the mobile terminal comprises a display device, wherein the detected animal parameters or evaluations based on the detected animal parameters are displayable on the display device.

## Revendications

1. Dispositif (10) pour l'enregistrement individuel de paramètres d'animaux, dans lequel le dispositif (10) comprend un ensemble capteur avec un ou plusieurs capteurs pour enregistrer les paramètres d'animaux, dans lequel le dispositif (10) peut être fixé à la tête et/ou au cou d'un animal (18), dans lequel l'ensemble capteur comprend un capteur de différence de pression et un dispositif de mesure du capteur de différence de pression peut être fixé dans au moins une ouverture nasale de l'animal, dans lequel le capteur de différence de pression est de préférence configuré pour mesurer une pression relative dans un écoulement de fluide de gaz respiratoires dans le nez de l'animal, **caractérisé en ce que** l'ensemble capteur comprend en outre un capteur pour mesurer une teneur en humidité dans l'écoulement de fluide, et
dans lequel des conclusions concernant un état de l'animal (18) peuvent être déduites des paramètres d'animaux enregistrés et/ou différents états de l'animal (18) peuvent être affichés avec une identification spécifique à l'état sur le dispositif (10).

2. Dispositif (10) selon la revendication 1
**caractérisé en ce que**
l'ensemble capteur comprend un ou plusieurs capteurs choisis dans un groupe comprenant un capteur gyroscopique, un accéléromètre, un capteur de température, un capteur GPS, un capteur NFC, un capteur RFID, un capteur de qualité de l'air, un capteur de composition chimique organique de l'air expiré par ledit animal et/ou un capteur pour la mesure de la fréquence cardiaque.

3. Dispositif (10) selon la revendication 1 ou 2
**caractérisé en ce que**
le dispositif (10) comprend une puce RFID pour reconnaître les animaux (18).

4. Dispositif (10) selon une ou plusieurs des revendications précédentes
**caractérisé en ce que**
le dispositif (10) comprend un capteur NFC pour reconnaître les animaux (18).

5. Dispositif (10) selon une ou plusieurs des revendications précédentes
**caractérisé en ce que**
le dispositif (10) est réalisé sous la forme d'un anneau nasal.

6. Dispositif (10) selon la revendication 5
**caractérisé en ce que**
le dispositif (10) comprend une construction annulaire, dans lequel la construction annulaire présente deux moitiés essentiellement symétriques, entre lesquelles la construction annulaire est interrompue en un point, dans lequel les deux moitiés de la construction annulaire sont chacune pourvues de mâchoires de maintien (22) aux extrémités, qui maintiennent le dispositif (10) dans le nez de l'animal (18) en appuyant contre une cloison nasale (12) de l'animal (18).

7. Dispositif (10) selon une ou plusieurs des revendications 1 à 4
**caractérisé en ce que**
le dispositif (10) est réalisé sous la forme d'un collier.

8. Dispositif (10) selon une ou plusieurs des revendications 1 à 4
**caractérisé en ce que**
le dispositif (10) est réalisé sous la forme d'un stent ou comprend un stent.

9. Dispositif (10) selon une ou plusieurs des revendications précédentes
**caractérisé en ce que**
le dispositif (10) comprend un moyen de fixation (24), dans lequel le moyen de fixation (24) est réalisé sous la forme d'une agrafe ou d'un fermoir.

10. Dispositif (10) selon une ou plusieurs des revendications précédentes
**caractérisé en ce que**
le dispositif (10) peut être fixé avec les moyens de fixation (24) à une aile nasale et/ou aux narines de l'animal (18).

11. Dispositif (10) selon une ou plusieurs des revendications précédentes
**caractérisé en ce que**
le dispositif (10) comprend une source lumineuse pour identifier les différents états de l'animal (18).

12. Dispositif (10) selon une ou plusieurs des revendications précédentes
**caractérisé en ce que**
les paramètres d'animaux enregistrés sont évalués dans le dispositif (10) lui-même.

13. Système d'enregistrement et d'évaluation de paramètres d'animaux, dans lequel le système comprend au moins un dispositif (10) selon une ou plusieurs des revendications précédentes, ainsi qu'un dispositif PC
**caractérisé en ce que**
les paramètres d'animaux enregistrés par l'au moins un dispositif (10) sont transmis au dispositif PC pour évaluation, dans lequel le dispositif PC est configuré pour transmettre des signaux au dispositif d'enregistrement des paramètres d'animaux en fonction des résultats d'évaluation, de sorte qu'un affichage visuel du dispositif est activé en fonction des signaux transmis.

14. Système pour l'identification individuelle d'animaux, le système comprenant au moins un dispositif (10) selon une ou plusieurs des revendications 1 à 12, ainsi qu'un dispositif PC et/ou un terminal mobile
**caractérisé en ce que**
des animaux individuels peuvent être sélectionnés sur le dispositif PC et/ou sur le terminal mobile, dans lequel le dispositif PC et/ou le terminal mobile sont configurés pour transmettre des signaux concernant les animaux sélectionnés au(x) dispositif(s) (10), de sorte qu'un affichage visuel du dispositif (10) est activé en fonction des signaux transmis.

15. Produit programme informatique pour la gestion d'un troupeau d'animaux
**caractérisé en ce que**
lorsqu'il est installé sur un dispositif (10) pour l'acquisition individuelle de paramètres d'animaux selon une ou plusieurs des revendications précédentes 1 à 12, il exécute les étapes suivantes :
a) évaluation de paramètres d'animaux enregistrés par l'ensemble capteur, dans lequel l'ensemble capteur comprend un ou plusieurs capteurs,
b) détermination d'un état de l'animal (18),
et de préférence également les étapes suivantes :
c) transmission d'un signal avec des informations sur l'état de l'animal au dispositif pour l'enregistrement individuel des paramètres d'animaux, dans lequel le signal transmis active un affichage visuel sur le dispositif (10),
d) affichage de l'état de l'animal (18) par l'affichage visuel,
et de préférence également l'étape supplémentaire suivante :
e) transmission d'un signal avec des informations sur l'état de l'animal (18) à un terminal mobile, dans lequel le terminal mobile comprend un dispositif d'affichage, dans lequel les paramètres d'animaux enregistrés ou des évaluations basées sur les paramètres d'animaux enregistrés peuvent être affichés sur le dispositif d'affichage.
